# EUROPEAN PATENT APPLICATION

(11) **EP 3 315 603 A1**
(43) Date of publication of application: **02.05.2018**
(21) Application number: 16872965.5
(22) Date of filing: 06.12.2016
(51) Int. Cl.: C12N 5/0775

(54) **STEM CELL CULTURING METHOD**

(30) Priority: 07.12.2015 JP 2015238349; 08.11.2016 JP 2016218144
(71) Applicant: Kintarocellspower Co., Ltd., Tokyo 141-0022 (JP)
(72) Inventor: GLADKOV, Alexei, Tokyo 141-0022 (JP)
(74) Representative: Ashton, Gareth Mark
(86) International application number: PCT/JP2016/086206
(87) International publication number: WO 2017/099067

(57) **Abstract**

[Problem] To provide a stem cell culturing method capable of preventing the proliferation of various stem cells and of culturing only specific types of stem cells. [Solution] A stem cell culturing method in which a second bone marrow aspirate 23 that is an intermediate layer is extracted from a first bone marrow aspirate 19 that is separated into layers; the second bone marrow aspirate 23 is cultured along with a culture medium and first stem cells are fixed on a bottom surface of a first culturing vessel; when a total surface area of the first stem cells reaches a first target ratio with respect to the bottom surface area of the first culturing vessel, the first stem cells are extracted from the first culturing vessel; top layer second stem cells are extracted from the first stem cells that are separated into layers and the second stem cells are cultured along with a culture medium, and the second stem cells are fixed on a bottom surface of a second culturing vessel; and, when a total surface area of the second stem cells reaches a second target ratio with respect to the bottom surface area of the second culturing vessel, the second stem cells are extracted from the second culturing vessel.

## Description

### TECHNICAL FIELD

The present invention relates to a stem cell culturing method for culturing stem cells by using a bone marrow aspirate collected from a donor.

### BACKGROUND ART

There is disclosed a stem cell culturing method having a medium for culturing stem cells and a laser irradiation means for activating the stem cells by irradiating an irradiation light of a low output carbon dioxide gas laser having an irradiation energy of exceeding 0 and 10 joule/cm² or less, a laser power density of 0.1 W/cm² or less, which is defocused to an irradiation energy of 0.1 or more and 2.5 joule/cm² or less to irradiate to the whole medium, wherein the stem cells are activated by irradiating the low output laser to activate them and thereafter a predetermined rest period is set in the stem cells to proliferate to a target number of proliferation. According to this stem cell culturing method, stem cells existing in tissues or cells thereof collected from human or non-human (animal) are activated and can be dramatically proliferated.

### PRIOR ART DOCUMENT

### PATENT DOCUMENT

Patent document 1: JP 2015-186465A

### SUMMARY OF THE INVENTION

### PROBLEMS TO BE SOLVED BY THE INVENTION

Since various stem cells are present in tissues or cells thereof collected from a donor, even if the stem cells existing in the tissues or cells are cultured, various stem cells are proliferated and only specific types of stem cells cannot be cultured. The stem cells are utilized for treatment, regenerative medicine or non-therapeutic applications of various diseases (cardiovascular and central nervous system diseases, etc.), but the cultured various stem cells have small therapeutic effect to various diseases or regenerative effect in regenerative medicine, low possibility of completely curing various diseases and low possibility of regenerating various tissues or various organs as compared with the case where it comprises only specific types of stem cells. In the above mentioned stem cell culturing method disclosed in Patent document 1, various stem cells are cultured and only specific types of stem cells cannot be cultured.

An object of the present invention is to provide a stem cell culturing method capable of preventing proliferation of various stem cells and of culturing only specific types of stem cells. Another object of the present invention is to provide a stem cell culturing method which can culture stem cells having large therapeutic effect to various diseases or regenerative effect in regenerative medicine, high possibility of completely curing various diseases and high possibility of regenerating various tissues or various organs.

### MEANS TO SOLVE THE PROBLEMS

Premise of the present invention to solve the above-mentioned problems is a stem cell culturing method for culturing a specific type of stem cells by using a first bone marrow aspirate collected from a donor.

The characteristic feature of the present invention in the above-mentioned premise is that the stem cell culturing method comprises a bone marrow aspirate separating step of separating a first bone marrow aspirate collected from a donor into layers, a bone marrow aspirate extracting step of extracting a second bone marrow aspirate positioned at an intermediate layer among the first bone marrow aspirate separated into layers in the bone marrow aspirate separating step, a stem cell first extracting step of injecting the second bone marrow aspirate extracted in the bone marrow aspirate extracting step and a predetermined culture medium into a first culturing vessel having a predetermined volume and a predetermined area of a bottom surface, statically allowing the first culturing vessel for a predetermined time to fix the first stem cells to the bottom surface of the first culturing vessel and proliferating the first stem cells, and when a total surface area of the first stem cells with respect to the bottom surface area of the first culturing vessel reached a first target ratio by expanding a planar shape of the first stem cells at the bottom surface of the first culturing vessel, extracting the first stem cells from the first culturing vessel, a stem cell centrifugation step of centrifuging the first stem cells extracted in the stem cell first extracting step into layers, a stem cell second extracting step of extracting the second stem cells positioned at an undermost layer after separating the first stem cells into layers in the stem cell centrifugation step, and a stem cell third extracting step of injecting the second stem cells extracted in the stem cell second extracting step and a predetermined culture medium into a second culturing vessel having a larger capacity and a larger bottom surface area of the bottom surface than those of the first culturing vessel, statically allowing the second culturing vessel for a predetermined time to fix the second stem cells to the bottom surface of the second culturing vessel and proliferating the second stem cells, and when a total surface area of the second stem cells with respect to the bottom surface area of the second culturing vessel reached a second target ratio by expanding a planar shape of the second stem cells at the bottom surface of the second culturing vessel, extracting the second stem cells from the second culturing vessel.

As an example of the present invention, the stem cell culturing method comprises a deformation first observation step of injecting the second bone marrow aspirate extracted in the bone marrow aspirate extracting step and the culture medium into the first culturing vessel, then, observing deformation of the first stem cells in the first culturing vessel from an initial planar shape while statically allowing it for a predetermined time in a state of inclining the first culturing vessel at a predetermined angle with predetermined time intervals, and a total surface area first observation step of injecting a new culture medium into the first culturing vessel while discharging the culture medium in the first culturing vessel by judging the first stem cells be fixed to the bottom surface of the first culturing vessel when the first stem cells are deformed from the initial planar shape to a predetermined planar shape as a result of observation in the deformation first observation step, and observing a total surface area of the first stem cells fixed to the bottom surface of the first culturing vessel with respect to the bottom surface area of the first culturing vessel while statically allowing it for a predetermined time in a state of inclining the first culturing vessel at a predetermined angle with predetermined time intervals, and in the stem cell first extracting step, when the planar shape of the first stem cells is expanded by proliferating the first stem cells and the total surface area of the first stem cells with respect to the bottom surface area of the first culturing vessel reached the first target ratio as a result of observation in the total surface area first observation step, the first stem cells are extracted from the first culturing vessel.

As another example of the present invention, the stem cell culturing method comprises a deformation second observation step of injecting the second stem cells extracted in the stem cell second extracting step and the culture medium into the second culturing vessel, then, observing deformation of the second stem cells in the second culturing vessel from an initial planar shape while statically allowing it for a predetermined time in a state of inclining the second culturing vessel at a predetermined angle for a predetermined time with predetermined time intervals, and a total surface area second observation step of injecting a new culture medium into the second culturing vessel while discharging the culture medium in the second culturing vessel by judging the second stem cells be fixed to the bottom surface of the second culturing vessel when the second stem cells are deformed from the initial planar shape to a predetermined planar shape as a result of observation in the deformation second observation step, and observing a total surface area of the second stem cells fixed to the bottom surface of the second culturing vessel with respect to the bottom surface area of the second culturing vessel while statically allowing it for a predetermined time in a state of inclining the second culturing vessel at a predetermined angle with predetermined time intervals, and in a stem cell third extracting step, as a result of observation in the total surface area second observation step, when the planar shape of the second stem cells is expanded by proliferating the second stem cells and the total surface area of the second stem cells with respect to the bottom surface area of the second culturing vessel reached the second target ratio, the second stem cells are extracted from the second culturing vessel.

As another example of the present invention, in the bone marrow aspirate separating step, 2 to 3 cc of the first bone marrow aspirate are collected from a donor, the 2 to 3 cc of the first bone marrow aspirate are injected into a separating vessel extending in the vertical direction, and the separating vessel is statically allowed at substantially the same temperature as the body temperature for a predetermined time to separate the first bone marrow aspirate in the separating vessel into layers in the vertical direction, and in the bone marrow aspirate extracting step, the second bone marrow aspirate positioned at an intermediate layer in the first bone marrow aspirate separated into layers in the separating vessel is extracted.

As another example of the present invention, a capacity of the first culturing vessel is about 20 to 30 cc, an initial planar shape of the first stem cells is substantially circular, a planar shape of the first stem cells after deformation is a flat shape in which the first stem cells are extended in irregular in one direction with substantially the circular as a core, and in the deformation first observation step, deformation of the first stem cells in the first culturing vessel from an initial planar shape is observed during 12 to 24 hours with intervals of about 1 to 2 hours while statically allowing the first culturing vessel at substantially the same temperature as the body temperature for 12 to 24 hours, and when the first stem cells are deformed in an irregular flat shape, the first stem cells are judged to be fixed to the bottom surface of the first culturing vessel.

As another example of the present invention, in the total surface area first observation step, the total surface area of the first stem cells fixed to the bottom surface of the first culturing vessel with respect to the bottom surface area of the first culturing vessel is observed during 36 to 48 hours with intervals of about 1 to 2 hours while statically allowing the first culturing vessel at substantially the same temperature as the body temperature for 36 to 48 hours.

As another example of the present invention, the first target ratio of the total surface area of the first stem cells with respect to the bottom surface area of the first culturing vessel is 70 to 80%.

As another example of the present invention, in the stem cell centrifugation step, the first stem cells is injected into the separating vessel, the separating vessel is located in a centrifugal separator to centrifuge the first stem cells, and in the stem cell second extracting step, the first stem cells are centrifuged into layers in the separating vessel, and then, the second stem cells located at the undermost layer are extracted.

As another example of the present invention, a capacity of the second culturing vessel is about 40 to 60 cc, an initial planar shape of the second stem cells is substantially circular, a planar shape of the first stem cells after deformation of the second stem cells is a flat shape in which the second stem cells are extended in irregular in one direction with substantially the circular as a core, and in the deformation second observation step, deformation of the second stem cells in the second culturing vessel from an initial planar shape is observed during 36 to 48 hours with intervals of about 1 to 2 hours while statically allowing the second culturing vessel at substantially the same temperature as the body temperature for 36 to 48 hours, and when the second stem cells are deformed in an irregular flat shape, the second stem cells are judged to be fixed to the bottom surface of the second culturing vessel.

As another example of the present invention, in the total surface area second observation step, the total surface area of the second stem cells fixed to the bottom surface of the second culturing vessel with respect to the bottom surface area of the second culturing vessel is observed during the 36 to 48 hours with intervals of about 1 to 2 hours while statically allowing the second culturing vessel at substantially the same temperature as the body temperature for 36 to 48 hours.

As another example of the present invention, the second target ratio of the total surface area of the second stem cells with respect to the bottom surface area of the second culturing vessel is 88 to 92%.

As another example of the present invention, the first and the second stem cells are mesenchymal stem cells.

### EFFECTS OF THE INVENTION

According to the stem cell culturing method in accordance with the present invention, the second bone marrow aspirate positioned at an intermediate layer among the first bone marrow aspirate separated into layers is extracted, the second bone marrow aspirate is cultured with a culture medium to fix the first stem cells to the bottom surface of the first culturing vessel and the first stem cells are proliferated. When the total surface area of the first stem cells reached the first target ratio with respect to the bottom surface area of the first culturing vessel, the first stem cells are extracted from the first culturing vessel, the second stem cells positioned at the undermost layer among the first stem cells centrifuged into layers are extracted, the second stem cells are cultured with the culture medium to fix the second stem cells to the bottom surface of the second culturing vessel and the second stem cells are proliferated. When the total surface area of the second stem cells reached the second target ratio with respect to the bottom surface area of the second culturing vessel, the second stem cells are extracted from the second culturing vessel. Thus, by extracting the specific second bone marrow aspirate from the first bone marrow aspirate separated into layers, and by extracting the specific second stem cells from the first stem cells separated into layers, proliferation of various stem cells can be prevented and only specific types of stem cells (the second stem cells) which are an object to be produced can be cultured, whereby pure (genuine) stem cells from which unnecessary stem cells had been removed can be produced. The stem cell culturing method can culture only specific types of stem cells so that stem cells having great therapeutic effect to various diseases and regenerative effect in regenerative medicine, and having high possibility of completely curing various diseases as well as high possibility of regenerating various tissues or various organs can be cultured.

In the stem cell culturing method in which, after injecting the extracted second bone marrow aspirate and the culture medium into the first culturing vessel, deformation of the first stem cells in the first culturing vessel from an initial planar shape is observed while statically allowing it for a predetermined time in a state of inclining the first culturing vessel at a predetermined angle with predetermined time intervals, and when the first stem cells are deformed from the initial planar shape to a predetermined planar shape as a result of observation of the deformation of the first stem cells, the first stem cells are judged to be fixed to the bottom surface of the first culturing vessel, and a new culture medium is injected into the first culturing vessel while discharging the culture medium in the first culturing vessel, the total surface area of the first stem cells fixed to the bottom surface of the first culturing vessel with respect to the bottom surface area of the first culturing vessel is observed while statically allowing it for a predetermined time in a state of inclining the first culturing vessel at a predetermined angle with predetermined time intervals, and when the total surface area of the first stem cells reached the first target ratio with respect to the bottom surface area of the first culturing vessel by proliferating the first stem cells to expand the planar shape of the first stem cells as a result of observation of the total surface area, then the first stem cells are extracted from the first culturing vessel, the first stem cells can be firmly fixed to the bottom surface of the first culturing vessel by statically allowing it in the state of inclining the first culturing vessel at a predetermined angle for a predetermined time, and after fixing the first stem cells to the bottom surface of the first culturing vessel, a new culture medium is injected into the first culturing vessel while discharging the culture medium in the first culturing vessel as well as it is statically allowed in the state of inclining the first culturing vessel at a predetermined angle for a predetermined time, whereby proliferation of the first stem cells can be certainly promoted. In the stem cell culturing method, fixation of the first stem cells to the bottom surface of the first culturing vessel can be accurately confirmed by observing deformation of the first stem cells in the first culturing vessel from an initial planar shape, and the degree of attainment of the total surface area of the first stem cells to the first target ratio can be accurately confirmed. In the stem cell culturing method, the first stem cells can be surely fixed and proliferated in the first culturing vessel as well as the degree of attainment of the total surface area of the first stem cells to the first target ratio can be accurately confirmed, so that only the first stem cells containing no other stem cells can be cultured, and only specific types of stem cells (the second stem cells) which are an object to be produced can be cultured while preventing proliferation of various stem cells.

The stem cell culturing method in which, after injecting the extracted second stem cells and the culture medium into the second culturing vessel, deformation of the second stem cells in the second culturing vessel from an initial planar shape is observed while statically allowing it in a state of inclining the second culturing vessel at a predetermined angle for a predetermined time with predetermined time intervals, and when the second stem cells are deformed from the initial planar shape to a predetermined planar shape as a result of observation of the deformation of the second stem cells, the second stem cells are judged to be fixed to the bottom surface of the second culturing vessel, and a new culture medium is injected into the second culturing vessel while discharging the culture medium in the second culturing vessel, the total surface area of the second stem cells fixed to the bottom surface of the second culturing vessel with respect to the bottom surface area of the second culturing vessel is observed while statically allowing it in a state of inclining the second culturing vessel at a predetermined angle for a predetermined time with predetermined time intervals, and as a result of observation of the total surface area, when the total surface area of the second stem cells reached the second target ratio with respect to the bottom surface area of the second culturing vessel by proliferating the second stem cells to expand the planar shape of the second stem cells, then the second stem cells are extracted from the second culturing vessel, can firmly fix the second stem cells to the bottom surface of the second culturing vessel by statically allowing it in a state of inclining the second culturing vessel at a predetermined angle for a predetermined time, and can certainly promote proliferation of the second stem cells by injecting a new culture medium into the second culturing vessel while discharging the culture medium in the second culturing vessel after fixing the second stem cells to the bottom surface of the second culturing vessel as well as statically allowing the second culturing vessel in a state of inclining at a predetermined angle for a predetermined time. In the stem cell culturing method, fixation of the second stem cells to the bottom surface of the second culturing vessel can be accurately confirmed by observing deformation of the second stem cells in the second culturing vessel from an initial planar shape, and the degree of attainment of the total surface area of the 21st stem cells to the second target ratio can be accurately confirmed. In the stem cell culturing method, the second stem cells can be surely fixed and proliferated in the second culturing vessel as well as the degree of attainment of the total surface area of the second stem cells to the second target ratio can be accurately confirmed, so that only the second stem cells containing no other stem cells can be cultured, and only specific types of stem cells (the second stem cells) which are an object to be produced can be cultured while preventing proliferation of various stem cells.

In the stem cell culturing method in which 2 to 3 cc of the above-mentioned first bone marrow aspirate are collected from a donor, the 2 to 3 cc of the first bone marrow aspirate are injected into the separating vessel extending in the vertical direction, and the separating vessel is statically allowed at substantially the same temperature as the body temperature for a predetermined time to separate the first bone marrow aspirate in the separating vessel into layers in the vertical direction, and the second bone marrow aspirate positioned at an intermediate layer in the first bone marrow aspirate separated into layers in the separating vessel is extracted, specific second bone marrow aspirate can be certainly extracted from the first bone marrow aspirate by statically allowing the first bone marrow aspirate containing various stem cells at substantially the same temperature as the body temperature for a predetermined time to separate it into layers in the vertical direction, and unnecessary stem cells contained in the first bone marrow aspirate can be removed as well as only specific types of stem cells (the second stem cells) which are an object to be produced can be cultured. Although 150 to 200 cc of bone marrow aspirate are generally needed for the culture of the stem cells, in the stem cell culturing method, 2 to 3 cc of bone marrow aspirate may be collected from the donor, and specific types of stem cells (the second stem cells) can be cultured with a small amount of the bone marrow aspirate, so that a sampling time of the bone marrow aspirate can be drastically shortened and the bone marrow aspirate can be collected at a low cost, and the burden on the donor at the time of collecting the bone marrow aspirate can be minimized.

In the stem cell culturing method in which a capacity of the first culturing vessel is about 20 to 30 cc, an initial planar shape of the first stem cells is substantially circular, a planar shape of the first stem cells after deformation is a flat shape in which the first stem cells are extended in irregular in one direction with substantially the circular as a core, deformation of the first stem cells in the first culturing vessel from an initial planar shape is observed during 12 to 24 hours with intervals of about 1 to 2 hours while statically allowing the first culturing vessel at substantially the same temperature as the body temperature for 12 to 24 hours, and when the first stem cells are deformed in an irregular flat shape, then the first stem cells are judged to be fixed to the bottom surface of the first culturing vessel, when a large culture vessel having a capacity exceeding 30 cc is used at the time of fixing the first stem cells, it becomes difficult for the first stem cells to fix on the bottom of the vessel and proliferation of the first stem cells slows down, but by using the first culturing vessel having the above-mentioned capacity, the first stem cells can be fixed to the bottom surface of the first culturing vessel quickly and easily, and the first stem cells can be rapidly proliferated in the first culturing vessel. In the stem cell culturing method, since deformation of the first stem cells in the first culturing vessel is observed from an initial planar shape during 12 to 24 hours with intervals of about 1 to 2 hours while statically allowing the first culturing vessel at substantially the same temperature as the body temperature for 12 to 24 hours, deformation of the first stem cells is never overlooked, fixation of the first stem cells to the bottom surface of the first culturing vessel can be accurately confirmed, and after confirming the fixation of the first stem cells, by injecting a new culture medium into the first culturing vessel while discharging the culture medium in the first culturing vessel, proliferation of the first stem cells can be certainly promoted. In the stem cell culturing method, the first stem cells are judged to be fixed to the bottom surface of the first culturing vessel when the first stem cells are deformed in a flat shape extended in irregular in one direction with substantially the circular as a core, fixation of the first stem cells to the bottom surface of the first culturing vessel is never overlooked, and the fixation of the first stem cells to the bottom surface of the first culturing vessel can be accurately grasped by the change in the planar shape of the first stem cells.

In the stem cell culturing method in which the total surface area of the first stem cells fixed to the bottom surface of the first culturing vessel with respect to the bottom surface area of the first culturing vessel is observed during 36 to 48 hours with intervals of about 1 to 2 hours while statically allowing the first culturing vessel at substantially the same temperature as the body temperature for 36 to 48 hours, the total surface area of the first stem cells with respect to the bottom surface area of the first culturing vessel can be accurately confirmed by observing the total surface area of the first stem cells fixed to the bottom surface of the first culturing vessel with intervals of about 1 to 2 hours, and it can be certainly grasped that the total surface area of the first stem cells has reached the first target ratio with respect to the bottom surface area of the first culturing vessel. In the stem cell culturing method, the first stem cells can be extracted from the first culturing vessel while maintaining the activity of the first stem cells without mistaking the timing of the extraction of the first stem cells from the first culturing vessel.

In the stem cell culturing method in which the first target ratio of the total surface area of the first stem cells with respect to the bottom surface area of the first culturing vessel is 70 to 80%, the activity of the first stem cells is gradually lost when the first stem cells are proliferated exceeding the total surface area of the first stem cells of 80% to the bottom surface area of the first culturing vessel, the first stem cells are extracted from the first culturing vessel when the total surface area of the first stem cells reach 70 to 80% to the bottom surface area of the first culturing vessel, so that the activity of the first stem cells can be retained and the first stem cells can be proliferated with the state of retaining the activity.

In the stem cell culturing method in which the first stem cells are injected into the separating vessel, the separating vessel is located in a centrifugal separator to centrifuge the first stem cells, the first stem cells is centrifuged into layers in the separating vessel, and then, the second stem cells located at the undermost layer are extracted, the first stem cells containing unnecessary mongrel stem cells are separated into layers by centrifugation using a centrifugal separator and the second stem cells positioned at the undermost layer in the first stem cells centrifuged into layers are extracted, whereby the specific second stem cells can be certainly extracted from the first stem cells, and unnecessary stem cells can be removed from the first stem cells as well as only specific types of stem cells (the second stem cells) which are an object to be produced can be cultured.

In the stem cell culturing method in which a capacity of the second culturing vessel is about 40 to 60 cc, an initial planar shape of the second stem cells is substantially circular, a planar shape of the first stem cells after deformation of the second stem cells is a flat shape in which the second stem cells are extended in irregular in one direction with substantially the circular as a core, deformation of the second stem cells in the second culturing vessel from an initial planar shape is observed during 36 to 48 hours with intervals of about 1 to 2 hours while statically allowing the second culturing vessel at substantially the same temperature as the body temperature for 36 to 48 hours, and the second stem cells are judged to be fixed to the bottom surface of the second culturing vessel when the second stem cells are deformed in irregular flat shape, if a large culture vessel having a capacity exceeding 60 cc is used at the time of fixing the second stem cells, it becomes difficult for the second stem cells to fix on the bottom surface of the vessel and proliferation of the second stem cells slows down, but by using the second culturing vessel having the above-mentioned capacity, the second stem cells can be fixed to the bottom surface of the second culturing vessel quickly and easily, and the second stem cells can be rapidly proliferated in the second culturing vessel. In the stem cell culturing method, since deformation of the second stem cells in the second culturing vessel is observed from an initial planar shape during 36 to 48 hours with intervals of about 1 to 2 hours while statically allowing the second culturing vessel at substantially the same temperature as the body temperature for 36 to 48 hours, deformation of the second stem cells is never overlooked, fixation of the second stem cells to the bottom surface of the second culturing vessel can be accurately confirmed, and after confirming the fixation of the second stem cells, by injecting a new culture medium into the second culturing vessel while discharging the culture medium in the second culturing vessel, proliferation of the second stem cells can be certainly promoted. In the stem cell culturing method, the second stem cells are judged to be fixed to the bottom surface of the second culturing vessel when the second stem cells are deformed in a flat shape extended in irregular in one direction with substantially the circular as a core, fixation of the second stem cells to the bottom surface of the second culturing vessel is never overlooked, and the fixation of the second stem cells to the bottom surface of the second culturing vessel can be accurately grasped by the change in the planar shape of the second stem cells.

In the stem cell culturing method in which the total surface area of the second stem cells fixed to the bottom surface of the second culturing vessel with respect to the bottom surface area of the second culturing vessel is observed during 36 to 48 hours with intervals of about 1 to 2 hours while statically allowing the second culturing vessel at substantially the same temperature as the body temperature for 36 to 48 hours, the total surface area of the second stem cells with respect to the bottom surface area of the second culturing vessel can be accurately confirmed by observing the total surface area of the second stem cells fixed to the bottom surface of the second culturing vessel with intervals of about 1 to 2 hours, and it can be certainly grasped that the total surface area of the second stem cells has reached the second target ratio with respect to the bottom surface area of the second culturing vessel. In the stem cell culturing method, the second stem cells can be extracted from the second culturing vessel while maintaining the activity of the second stem cells without mistaking the timing of the extraction of the second stem cells from the second culturing vessel.

In the stem cell culturing method in which the second target ratio of the total surface area of the second stem cells with respect to the bottom surface area of the second culturing vessel is 88 to 92%, the activity of the second stem cells is gradually lost when the second stem cells are proliferated exceeding the total surface area of the second stem cells of 92% to the bottom surface area of the second culturing vessel, the second stem cells are extracted from the second culturing vessel when the total surface area of the second stem cells reaches 88 to 92% to the bottom surface area of the second culturing vessel, so that the activity of the second stem cells can be retained and the second stem cells having high activity can be produced. In the stem cell culturing method, only specific types of stem cells (the second stem cells) having high activity can be cultured so that the stem cells having great therapeutic effect to various diseases and regenerative effect in regenerative medicine and having high possibility of completely curing various diseases as well as high possibility of regenerating various tissues or various organs can be cultured.

In the stem cell culturing method in which the first and the second stem cells are mesenchymal stem cells, by extracting the specific second bone marrow aspirate from the first bone marrow aspirate separated into layers and by extracting the specific second mesenchymal stem cells from the first mesenchymal stem cells separated into layers, unnecessary mongrel mesenchymal stem cells are removed and proliferation of various mesenchymal stem cells can be prevented, whereby only specific types of mesenchymal stem cells (the second mesenchymal stem cells) which are an object to be produced can be cultured. The stem cell culturing method can culture only specific types of mesenchymal stem cells so that the mesenchymal stem cells having great therapeutic effect to various diseases and regenerative effect in regenerative medicine and having high possibility of completely curing various diseases as well as high possibility of regenerating various tissues or various organs can be cultured.

### BRIEF DESCRIPTION OF THE DRAWINGS

Fig. 1 is a schematic configuration diagram of a stem cell culture system shown as an example;
Fig. 2 is an explanation drawing showing an example of a bone marrow aspirate separation step;
Fig. 3 is an explanation drawing of a bone marrow aspirate separating step continued from Fig. 2;
Fig. 4 is an explanation drawing of a bone marrow aspirate separating step continued from Fig. 3;
Fig. 5 is an explanation drawing showing an example of a deformation first observation step;
Fig. 6 is a side view of a first flat culture vessel;
Fig. 7 is a partial enlarged view showing an example of a planar shape of first mesenchymal stem cells;
Fig. 8 is a partial enlarged view showing another example of a planar shape of first mesenchymal stem cells;
Fig. 9 is a partial enlarged view showing another example of a planar shape of first mesenchymal stem cells;
Fig. 10 is an explanation drawing showing an example of a stem cell centrifugation step;
Fig. 11 is an explanation drawing showing an example of a stem cell second extracting step;
Fig. 12 is an explanation drawing showing an example of a deformation second observation step;
Fig. 13 is a side view of a second flat culture vessel;
Fig. 14 is a partial enlarged view showing an example of a planar shape of second mesenchymal stem cells;
Fig. 15 is a partial enlarged view showing another example of a planar shape of second mesenchymal stem cells;
Fig. 16 is a partial enlarged view showing another example of a planar shape of second mesenchymal stem cells; and
Fig. 17 is a drawing showing an example of preservation of second mesenchymal stem cells.

### EMBODIMENTS TO CARRY OUT THE INVENTION

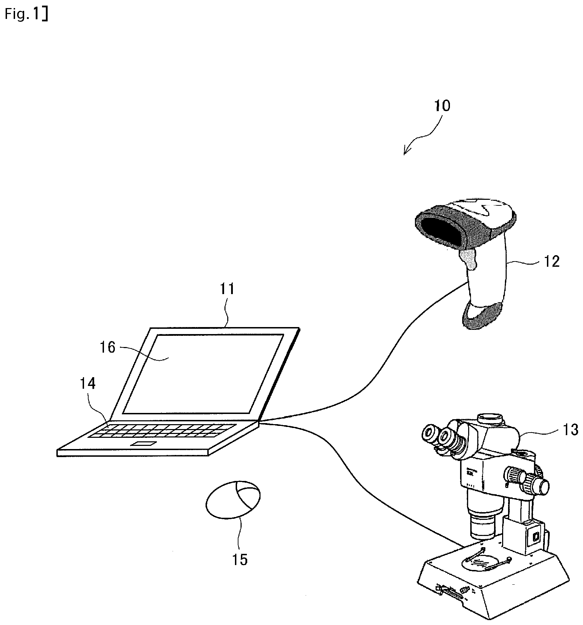

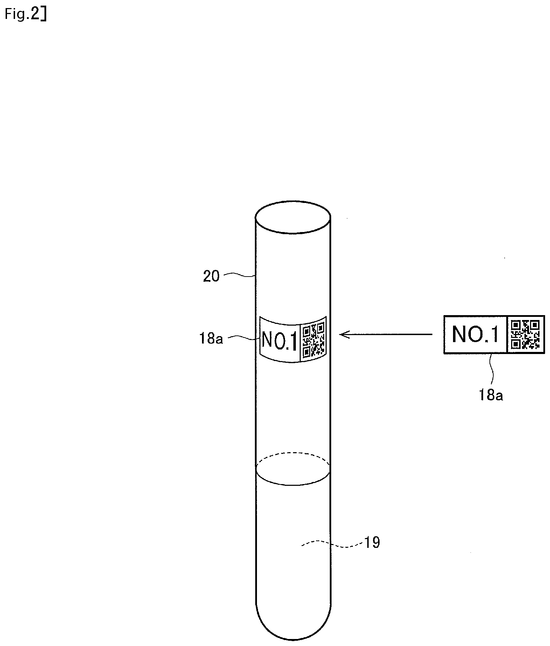

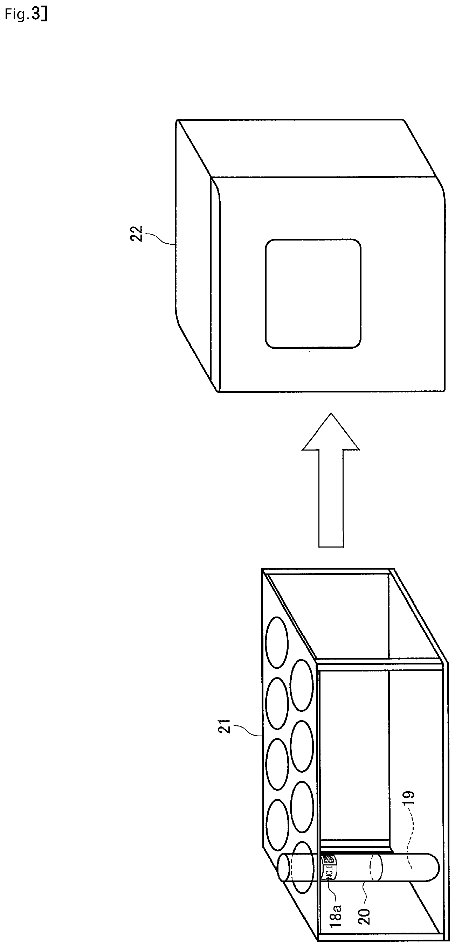

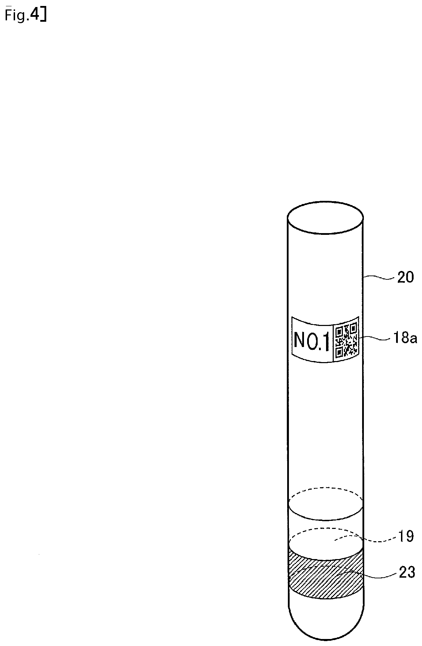

By referring to the attached drawings such as Fig. 1 which is a schematic configuration diagram of a stem cell culture system 10 shown as an example, etc., details of the stem cell culturing method according to the present invention are explained as follows. Incidentally, Fig. 2 is an explanation drawing showing an example of a bone marrow aspirate separation step, and Fig. 3 is an explanation drawing of the bone marrow aspirate separating step continued from Fig. 2. Fig. 4 is an explanation drawing of the bone marrow aspirate separating step continued from Fig. 3.

The stem cell culturing method utilizes a first bone marrow aspirate collected from multiple donors (human), and by subjecting to a bone marrow aspirate separating step, a bone marrow aspirate extracting step, a deformation first observation step), a total surface area first observation step, a stem cell first extracting step, a stem cell centrifugation step, a stem cell second extracting step, a deformation second observation step, a total surface area second observation step and a stem cell third extracting step, specific types of a single kind of mesenchymal stem cells are cultured (produced) in a plural kinds of mesenchymal stem cells contained in a first bone marrow aspirate 19.

The stem cell culturing system 10 is formed by a computer 11, a barcode reader 12 and an electron microscope 13. The computer 11 has a central processing unit (CPU or virtual CPU), a memory storage unit (memory or virtual memory) and a mass storage area (a hard disk or a virtual hard disk, etc.), and operated by a physical OS (operating system) or a virtual OS (virtual operating system). To the computer 11 are connected an input device such as a keyboard 14 and a mouse 15, etc., and an output device such as a display 16 and a printer (not shown in the drawing), etc., through an interface (wireless or wired).

In the stem cell culturing system 10, donor data (donor identifiable information) are managed by using the QR code (Registered Trademark). In the donor data, there are name, address, telephone number, date of birth, gender, blood type, height, weight, an e-mail address, etc., of the donor. In the system 10, the QR code is used as a two-dimensional code, and in addition to the QR code, a matrix system SP code, vericode (VeriCode), maxicode (MaxiCode), CP code, DataMatrix, Code1, AztecCode, intacta code and card e can be used. Incidentally, the two-dimensional code used in the system 10 includes all those to be developed in the future. Also, the donor data (donor identifiable information) can be managed by an IC tag (IC chip).

In the bone marrow aspirate separating step, the first bone marrow aspirate 19 collected from a donor is separated into layers. In the collection of the bone marrow aspirate, 2 to 3 cc (2 to 3 ml) of the first bone marrow aspirate 19 is collected from the sternum or iliac bone (pelvis) of the donor. The first bone marrow aspirate 19 is collected by "bone marrow aspiration" (mark) that local anesthesia is applied to the donor and then the bone marrow is punctured and the bone marrow aspirate (bone marrow blood) is aspirated. At the same time as collecting the first bone marrow aspirate 19, a person in charge such as a doctor, a nurse and a researcher, etc., activates the system 10 in the computer 11 and uses the input device such as the keyboard 14 and the mouse 15, etc., to input the donor data to the computer 11.

The computer 11 generates a unique donor identifier that specifies each donor every time the donor data is inputted (every time the first bone marrow aspirate 19 is collected from the donor) and stores (memorizes) in the storage area in the state that the donor data are associated with the donor identifier (donor data storage means). The computer 11 converts the input donor data into a QR code (two-dimensional code) by a two-dimensional code writer function (two-dimensional code (QR code) converting means), outputs the first to fourth code sheets 18a to 18d on which the QR code is printed (two-dimensional code (QR code) output means), and stores (memorizes) to the storage area in the state that the QR code are associated with the donor identifier which specifies each donor (two-dimensional code (QR code) storage means).

Incidentally, in the QR code printed on the first code sheet 18a on which NO1 has been printed, the number 1 up to the bone marrow aspirate separating step and the bone marrow aspirate extracting step is stored, and in the QR code printed on the second code sheet 18b on which NO2 has been printed, the number 2 up to the deformation first observation step, the total surface area first observation step and the stem cell first extracting step is stored. In the QR code printed on the third code sheet 18c on which NO3 has been printed, the number 3 up to the stem cell centrifugation step and the stem cell second extracting step is stored, and in the QR code printed on the fourth code sheet 18d on which NO4 has been printed, the number 4 up to the deformation second observation step, the total surface area second observation step and the stem cell third extracting step is stored. The computer 11 compares the donor data stored in the storage area with the donor data indicated by the QR code read by the barcode reader 12 for each step of from the bone marrow aspirate separating step to the stem cell third extracting step.

As shown in Fig. 2, 2 to 3 cc of the first bone marrow aspirate 19 collected from the donor are injected (accommodated) into the glass test tube 20 (the separating vessel) extending in the vertical direction. Incidentally, in 2 to 3 cc of the first bone marrow aspirate 19, 0.5 to 1 ml (about 5×10⁷ (cells/ml)) of a plural kinds of mesenchymal stem cells is contained. A first code sheet 18a is adhered to the outer peripheral surface of the glass test tube 20 into which the first bone marrow aspirate 19 is injected. As shown in Fig. 3, the glass test tube 20 into which the first bone marrow aspirate 19 has been injected is set in a test tube stand 21, and accommodated in a thermostat chamber 22 with the test tube stand 21.

A person in charge such as a doctor, a nurse and a researcher, etc., sticks the first code sheet 18a on which the QR code has been printed on the outer circumferential surface of the glass test tube 20, and then, causes the barcode reader 12 to read the QR code of the glass test tube 20. The barcode reader 12 is connected to the computer 11 via an interface (wired or wireless). The barcode reader 12 transmits the read QR code to the computer 11.

The computer 11 extracts the number 1 and the donor data indicated by the QR code transmitted from the barcode reader 12 from the storage area and reads them into the cache memory, and displays a process first display screen (not shown in the drawing) on a display 16. In the process first display screen, the number 1 and the donor data are displayed, and at the same time, a bone marrow aspirate separation button, a bone marrow aspirate extraction button, a deformation first observation button, a total surface area first observation button, a stem cell first extraction button, a stem cell centrifugation button, a stem cell second extraction button, a deformation second observation button, a total surface area second observation button, a stem cell third extraction button and a logout button are displayed. When culture of the stem cells is not carried out, the logout button is to be clicked. When the logout button is clicked, the system 10 is stopped in the computer 11.

After clicking the bone marrow aspirate separation button displayed on the display 16, the person in charge injects the first bone marrow aspirate 19 into the glass test tube 20 from a syringe, and the glass test tube 20 into which the first bone marrow aspirate 19 has been injected is inserted (set) into the test tube stand 21. As shown in Fig. 3, the person in charge accommodates the test tube stand 21 in the thermostat chamber 22, and the glass test tube 20 into which the first bone marrow aspirate 19 has been injected is statically allowed (leave it quietly without moving it) in the thermostat chamber 22 for a predetermined time (about 2 hours). The temperature inside the thermostat chamber 22 is maintained at about 36 to 37°C which is substantially the same as the body temperature. The computer 11 displays the number 1 indicated by the QR code printed on the first code sheet 18a and the donor data on the display 16, and also displays bone marrow aspirate separation on-going message and a bone marrow aspirate separation termination button on the display 16.

As shown in Fig. 4, by statically allowing the glass test tube 20 in the thermostat chamber 22 for a predetermined time (about 2 hours), the first bone marrow aspirate 19 injected into the test tube 20 is separated into layers with some layers (3 layers) in the test tube 20 in the vertical direction. 150 to 200 cc (150 to 200 ml) of the bone marrow aspirate are usually required for culturing stem cells, but in this stem cell culturing method, it is sufficient to collect only 2 to 3 cc of the first bone marrow aspirate 19 from the donor, and specific types of mesenchymal stem cells (the second mesenchymal stem cells 31) can be cultured (produced) with a small amount of the bone marrow aspirate 19, so that a sampling time of the bone marrow aspirate 19 can be drastically shortened, and the bone marrow aspirate 19 can be collected with a low cost and the burden on the donor at the time of collecting the bone marrow aspirate 19 can be minimized.

After the bone marrow aspirate separating step in which the first bone marrow aspirate 19 is separated into layers, the bone marrow aspirate extracting step is carried out. In the bone marrow aspirate extracting step, the second bone marrow aspirate 23 is extracted from the first bone marrow aspirate 19 separated into layers. After confirming that the first bone marrow aspirate 19 has separated into layers, the person in charge clicks the bone marrow aspirate separation termination button displayed on the display 16.

When the bone marrow aspirate separation termination button is clicked, the computer 11 displays a process second display screen (not shown in the drawing) on the display 16. In the process second display screen, the number 1 and the donor data are displayed, and at the same time, a bone marrow aspirate separation termination message, a bone marrow aspirate extraction button, a deformation first observation button, a total surface area first observation button, a stem cell first extraction button, a stem cell centrifugation button, a stem cell second extraction button, a deformation second observation button, a total surface area second observation button and a stem cell third extraction button are displayed.

The person in charge clicks the bone marrow aspirate extraction button on the process second display screen and makes the barcode reader 12 read the QR code of the glass test tube 20. When the QR code is transmitted from the barcode reader 12 to the computer 11, the computer 11 compares the donor data (the donor data read to the memory) displayed on the display 16 and the donor data indicated by the QR code read by the barcode reader 12, and when these donor data are matched, the number 1 and the donor data are displayed on the display 16, and at the same time, a bone marrow aspirate separation termination message, a bone marrow aspirate extraction on-going message and a bone marrow aspirate extraction termination button are displayed on the display. When these donor data are not matched, the computer 11 displays an error message and a culture stop message on the display 16.

The person in charge takes out the test tube stand 21 from the inside of the thermostat chamber 22, draws out the glass test tube 20 from the test tube stand 21, and extracts the second bone marrow aspirate 23 existing in a specific layer of the first bone marrow aspirate 19 separated into layers. The person in charge extracts (sucks) the second bone marrow aspirate 23 with a layer thickness of 3 to 4 mm positioned at the intermediate layer in the first bone marrow aspirate 19 separated into layers by utilizing a syringe (not shown in the drawing). Or else, the second bone marrow aspirate 23 with a layer thickness of 3 to 4 mm positioned at the intermediate layer in the first bone marrow aspirate 19 separated into layers is extracted (sucked) by utilizing a pipette (not shown in the drawing). In the stem cell culturing method, after separating the first bone marrow aspirate 19 containing various mesenchymal stem cells into layers (3 layers) in the vertical direction, a specific (intermediate) second bone marrow aspirate 23 can be certainly extracted from the first bone marrow aspirate 19 by utilizing a syringe or a pipette, and unnecessary mesenchymal stem cells contained in the first bone marrow aspirate 19 can be removed.

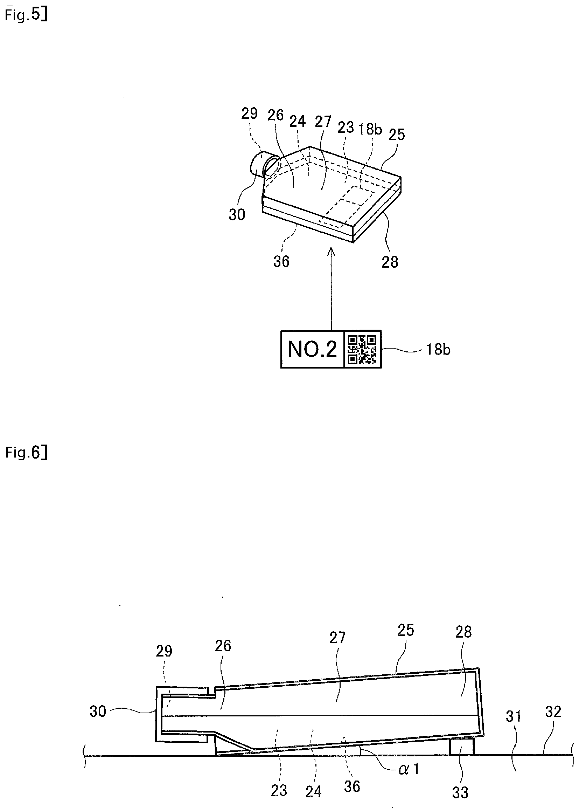

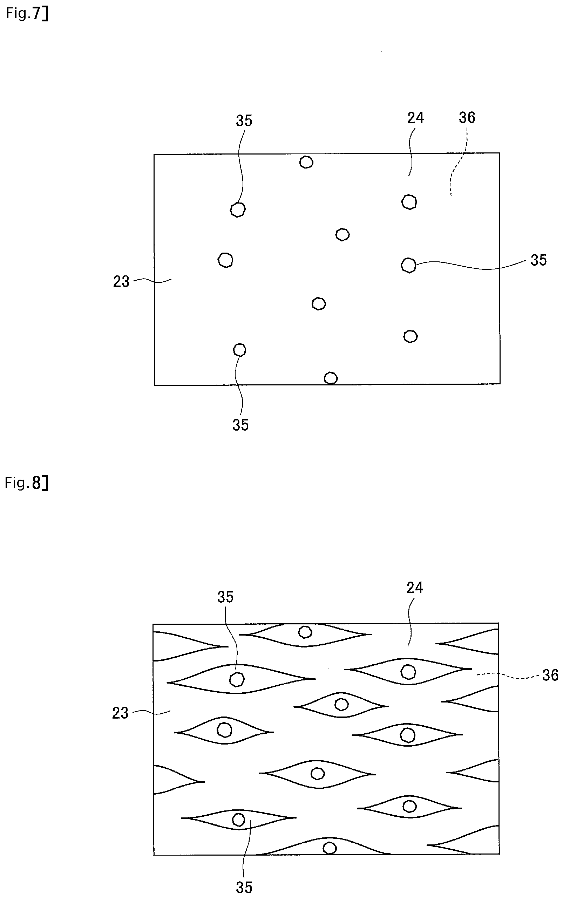

Fig. 5 is an explanation drawing showing an example of a deformation first observation step, and Fig. 6 is a side view of a first flat culture vessel 25 (the first culturing vessel). Fig. 7 is a partial enlarged view showing an example of a planar shape of the first mesenchymal stem cells 35, and Fig. 8 is a partial enlarged view showing another example of a planar shape of the first mesenchymal stem cells 35. Figs. 7 and 8 show enlarged views of the planar shape of the first mesenchymal stem cells 35 photographed by the electron microscope 13.

After the bone marrow aspirate extracting step in which specific second bone marrow aspirate 23 positioned at the intermediate layer is extracted from the first bone marrow aspirate 19, a deformation first observation step is carried out. In the deformation first observation step, the second bone marrow aspirate 23 and a culture medium 24 are injected (accommodated) into the first flat culture vessel 25 (the first culturing vessel) (the cell culture vessel), it is statically allowed (leave it quietly without moving it) for 12 to 24 hours while retaining inside the culture vessel 25 at substantially the same temperature as the body temperature (about 36 to 37°C), deformation of the first mesenchymal stem cells 35 (the first stem cells) contained in the second bone marrow aspirate 23 in the culture vessel 25 from an initial planar shape is observed by the electron microscope 13 during 12 to 24 hours with intervals of about 1 to 2 hours, and whether or not the stem cells 35 are fixed to the bottom surface 36 of the culture vessel 25 is judged. Onto the bottom surface 36 (the bottom wall outer surface) the first flat culture vessel 25 into which the second bone marrow aspirate 23 and the culture medium 24 are injected, the second code sheet 18b on which the QR code which specifies the donor is adhered.

The person in charge such as a doctor, a nurse and a researcher, etc., clicks the bone marrow aspirate extraction termination button displayed on the display 16 after extracting specific second bone marrow aspirate 23 form the first bone marrow aspirate 19. When the bone marrow aspirate extraction termination button is clicked, the computer 11 displays a process third display screen (not shown in the drawing) on the display 16. In the process third display screen, the number 1 and the donor data are displayed, and at the same time, a bone marrow aspirate separation termination message, a bone marrow aspirate extraction termination message, a deformation first observation button, a total surface area first observation button, a stem cell first extraction button, a stem cell centrifugation button, a stem cell second extraction button, a deformation second observation button, a total surface area second observation button and a stem cell third extraction button are displayed.

The person in charge adheres the second code sheet 18b on which the QR code is printed to the bottom surface 36 (the bottom wall outer surface) of the first flat culture vessel 25. Next, the deformation first observation button on the process third display screen is clicked to make the barcode reader 12 read the QR code of the culture vessel 25. When the QR code is transmitted from the barcode reader 12 to the computer 11, the computer 11 compares the donor data (the donor data read to the memory) displayed on the display 16 and the donor data indicated by the QR code read by the barcode reader 12, and when these donor data are matched, the number 2 and the donor data are displayed on the display 16, and at the same time, a bone marrow aspirate separation termination message, a bone marrow aspirate extraction termination message, a deformation first observation on-going message and a deformation first observation termination button are displayed on the display. When these donor data are not matched, the computer 11 displays an error message and a culture stop message on the display 16.

The first flat culture vessel 25 used in the deformation first observation step is a flat vessel made of transparent glass or transparent plastic and having a bottom surface 36 with a small capacity and a predetermined area and having a substantially square planar shape. The first flat culture vessel 25 has a top part 26, a bottom part 28, a central part 27 located between the top and bottom parts 26 and 28, and an injection port 29 formed at the top part 26. The injection port 29 is watertightly closed by a lid 30. The first flat culture vessel 25 has a capacity of about 20 to 30 cc (preferably 25 cc), and its bottom surface area is about 25 to 36 mm². The first flat culture vessel 25 has a length of one side thereof of 5 to 6 mm. Incidentally, as the first flat culture vessel 25, a flat vessel with a circular or elliptical in the planar shape and having a bottom surface 28 with a small capacity and a predetermined area may be used.

The person in charge removes the lid 30 from the injection port 29, injects (accommodates) the second bone marrow aspirate 23 aspirated by a syringe or a pipette into inside the culture vessel 25 from the injection port 29 of the culture vessel 25, and injects (accommodates) the culture medium 24 aspirated by the syringe or the pipette into inside the culture vessel 25 from the injection port 29 of the culture vessel 25, and closes the injection port 29 with the lid 30.

In the culture medium 24, a mineral salt solution to which penicillin (about 100 U/ml), amphotericin (about 100 ng/ml), streptomycin (about 100 mkg/ml), L-glutamine (about 2 to 4 ml) and 20% fetal bovine serum had been supplemented and amino acids are contained. The first mesenchymal stem cells 35 contained in the second bone marrow aspirate 23 injected into the first flat culture vessel 25 are cultured with the culture medium 24 while being fixed on the bottom surface 36 of the culture vessel 25 with the lapse of time, and gradually proliferate (differentiate) on the bottom surface 36 of the culture vessel 25 to form colonies.

After injecting the second bone marrow aspirate 23 and the culture medium 24 into the first flat culture vessel 25, the person in charge locates (sets) the culture vessel 25 in a sample holder 31 of the electron microscope 13. Incidentally, a spacer 33 is interposed between an upper surface 32 of the sample holder 31 of the electron microscope 13 and the bottom part 28 of the first flat culture vessel 25 so that the bottom part 28 of the culture vessel 25 is held in a lifted state by the spacer 33, and the culture vessel 25 is kept in a state of being inclined at a predetermined angle so that the bottom part 28 of the culture vessel 25 is on the top and the top part 26 (the injection port 29) of the culture vessel 25 is on the bottom. Also, a spacer 33 is interposed between the upper surface 32 of the sample holder 31 of the electron microscope 13 and the top part 26 of the first flat culture vessel 25, the top part 26 of the culture vessel 25 is held in a lifted state by the spacer 33, the culture vessel 25 may be kept in a state of being inclined at a predetermined angle so that the top part 26 of the culture vessel 25 is on the top and the bottom part 28 of the culture vessel 25 is on the bottom. An inclination angle α1 of the first flat culture vessel 25 with respect to the upper surface 32 of the sample holder 31 is in the range of 2 to 5°, preferably in the range of 2 to 3°.

In the stem cell culturing method, by inclining the first flat culture vessel 25 to the upper surface 32 of the sample holder 31 at the above-mentioned inclination angle, the second bone marrow aspirate 23 and the culture medium 24 are inclined on the side of the top part 26 (or on the side of the bottom part 28) of the culture vessel 25 in the first flat culture vessel 25, and on the side of the top part 26 (or the side of the bottom part 28) of the side of the culture vessel 25, the water pressures of the second bone marrow aspirate 23 and the culture medium 24 become large and the first mesenchymal stem cells 35 are concentrated on the side of the bottom surface 36 of the culture vessel 25, whereby the activities of the first mesenchymal stem cells 35 are increased and the first mesenchymal stem cells 35 can be easily and quickly fixed on the bottom surface 36 of the culture vessel 25.

The electron microscope 13 is connected to the computer 11 through an interface (wired or wireless). The electron microscope 13 has an image photographing function of photographing an enlarged image of a subject by an image pickup element and has an image transmission function of sending the enlarged image to the computer 11. The electron microscope 13 photographs an enlarged image of the planar shape of the first mesenchymal stem cells 35 contained in the second bone marrow aspirate 23 injected into the first flat culture vessel 25 with intervals of about 1 to 2 hours, and the photographed enlarged image of the planar shape of the stem cells 35 is transmitted to the computer 11 with intervals of about 1 to 2 hours. The image photographing interval and image transmission interval in the electron microscope 13 can be freely set within 1 to 2 hours by an input device such as the keyboard 14 and the mouse 15, etc.

The computer 11 stores (memorizes) the enlarged image of the planar shape of the first mesenchymal stem cells 35 and a photographing time transmitted from the electron microscope 13 in a storage area in the state of associating with the donor identifier (stem cells image storage means). The computer 11 displays the enlarged image of the planar shape of the first mesenchymal stem cells 35 and the photographing time transmitted from the electron microscope 13 on the display 16. The person in charge confirms (views) the enlarged image of the planar shape of the first mesenchymal stem cells 35 displayed on the display 16 during 12 to 24 hours with intervals of about 1 to 2 hours, and observes change in the planar shape of the stem cells 35 contained in the second bone marrow aspirate 23. Incidentally, the person in charge may directly observe the change in the planar shape of the first mesenchymal stem cells 35 from the observation window of the electron microscope 13 during 12 to 24 hours with intervals of about 1 to 2 hours.

An initial planar shape of the first mesenchymal stem cells 35 is substantially circular, and when the planar shape of the stem cells 35 is substantially circular, the stem cells 35 are not fixed on the bottom surface 36 (the bottom wall inner surface) of the first flat culture vessel 25 and proliferation (differentiation) of the stem cells 27 is not started. The planar shape after deformation of the first mesenchymal stem cell 27 is a flat shape in which the stem cells 27 is irregularly extended (expanded) in one direction (predetermined direction) with substantially the circular as a core before fixing, and the stem cells 27 is fixed on the bottom surface 36 (the bottom wall inner surface) of the first flat culture vessel 25 and proliferation (differentiation) of the stem cells 27 is started.

As shown in Fig. 6, when the enlarged image of the planar shape of the first mesenchymal stem cells 35 displayed on the display 16 is observed to be substantially circular as a result of observation in the deformation first observation step, the person in charge judges that the stem cells 35 are not fixed to the bottom surface 36 (the bottom wall inner surface) of the first flat culture vessel 25, and continuously observes changes in the planar shape of the stem cells 35 with intervals of about 1 to 2 hours. As shown in Fig. 7, when the planar shape of the first mesenchymal stem cells 35 displayed on the display 16 is deformed from substantially circular into an irregular flat shape with substantially the circular as a core as a result of observation in the deformation first observation step, the person in charge judges that the stem cells 35 is fixed to the bottom surface 35 of the first flat culture vessel 25.

If a large culture vessel having a capacity exceeding 30 cc and the bottom area exceeding 36 mm² at the time of fixing the first mesenchymal stem cell 35 is used, the stem cells 35 tend to be difficultly fixed to the bottom surface of the vessel and proliferation of the stem cells 35 becomes slow, but in the stem cell culturing method, by using the first flat culture vessel 25 having the above-mentioned capacity and the above-mentioned bottom surface area, the stem cells 35 can be easily fixed to the bottom surface 36 of the culture vessel 25 and the stem cells 27 can be quickly proliferated in the culture vessel 25.

In the stem cell culturing method, since deformation of the first mesenchymal stem cells 35 in the culture vessel 25 from an initial planar shape is observed during 12 to 24 hours with intervals of about 1 to 2 hours while statically allowing the first flat culture vessel 25 at substantially the same temperature as the body temperature for 12 to 24 hours, deformation of the stem cells 35 is never overlooked and fixation of the stem cells 35 to the bottom surface 36 of the culture vessel 25 can be accurately confirmed.

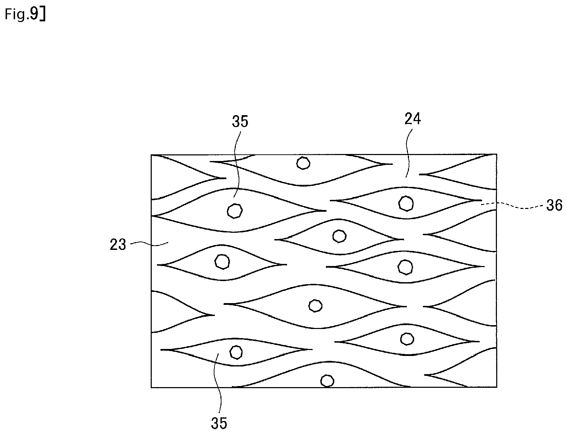

Fig. 9 is a partial enlarged view showing another example of a planar shape of the first mesenchymal stem cells 35. Fig. 9 shows the enlarged image of the planar shape of the first mesenchymal stem cells 35 photographed by the electron microscope 13. As a result of observation in the deformation first observation step, the first mesenchymal stem cells 35 (the first stem cells) is deformed from a substantially circular shape (an initial planar shape) to an irregular flat shape with substantially the circular as a core, and after the deformation first observation step confirming fixation of the stem cells 35 to the bottom surface 36 of the first flat culture vessel 25 (the first culturing vessel), the total surface area first observation step is carried out.

In the total surface area first observation step, the culture medium 24 injected into the first flat culture vessel 25 is discharged from the culture vessel 25, and a new culture medium 24 is injected (accommodated) into the culture vessel 25. Next, the total surface area of the first mesenchymal stem cells 35 fixed to the bottom surface 36 of the culture vessel 25 with respect to the bottom surface area of the culture vessel 25 is observed by the electron microscope 13 during 36 to 48 hours with intervals of about 1 to 2 hours while statically allowing (leaving it quietly without moving it) the first flat culture vessel 25 at substantially the same temperature as the body temperature (about 37°C) for 36 to 48 hours, and it is judged whether or not the total surface area of the stem cells 35 reached the first target ratio with respect to the bottom surface area of the culture vessel 25. The first target ratio of the total surface area of the first mesenchymal stem cells 35 with respect to the bottom surface area of the first flat culture vessel 25 is 70 to 80% (70 to 80% confluent).

The person in charge such as a doctor, a nurse and a researcher, etc., clicks the deformation first observation termination button displayed on the display 16 after confirming fixation of the first mesenchymal stem cells 35 contained in the second bone marrow aspirate 23 to the bottom surface 36 of the first flat culture vessel 25. When the deformation first observation termination button is clicked, the computer 11 displays a process fourth display screen (not shown in the drawing) on the display 16. In the process fourth display screen, the number 2 and the donor data are displayed, and at the same time, a bone marrow aspirate separation termination message, a bone marrow aspirate extraction termination message, a deformation first observation termination message, a total surface area first observation button, a stem cell first extraction button, a stem cell centrifugation button, a stem cell second extraction button, a deformation second observation button, a total surface area second observation button and a stem cell third extraction button are displayed.

The person in charge clicks a stem cells second observation button on the process fourth display screen, and the first flat culture vessel 25 is removed from the sample holder 31 of the electron microscope 16 and the QR code of the culture vessel 25 is read by the barcode reader 12. When the QR code is transmitted from the barcode reader 12 to the computer 11, the computer 11 compares the donor data (the donor data read to the memory) displayed on the display 16 and the donor data indicated by the QR code read by the barcode reader 12, and when these donor data are matched, the number 2 and the donor data are displayed on the display 16, and at the same time, a bone marrow aspirate separation termination message, a bone marrow aspirate extraction termination message, a deformation first observation termination message, a total surface area first observation on-going message and a total surface area first observation termination button are displayed on the display 16. When these donor data are not matched, the computer 11 displays an error message and a culture stop message on the display 16.

The person in charge discharges the culture medium 24 injected into the first flat culture vessel 25 in the deformation first observation step from the culture vessel 25 by utilizing a syringe or a pipette, and injects (accommodates) a new culture medium 24 aspirated by a syringe or a pipette into the culture vessel 25. The new culture medium 24 is the same as that injected in the deformation first observation step. The person in charge locates (sets) the culture vessel 25 at the sample holder 31 of the electron microscope 13 after injecting the new culture medium 24 into the first flat culture vessel 25.

Incidentally, a spacer 33 is interposed between the upper surface 32 of the sample holder 31 of the electron microscope 13 and the bottom part 28 of the first flat culture vessel 25, the bottom part 28 of the culture vessel 25 is held in a lifted state by the spacer 33, and the culture vessel 25 is kept in a state of being inclined at a predetermined angle so that the bottom part 28 of the culture vessel 25 is on the top and the top part 26 (the injection port 29) of the culture vessel 25 is on the bottom (see Fig. 6). Also, the spacer 33 is interposed between the upper surface 32 of the sample holder 31 of the electron microscope 13 and the top part 26 of the first flat culture vessel 25, the top part 26 of the culture vessel 25 is held in a lifted state by the spacer 33, and the culture vessel 25 may be kept in a state of being inclined at a predetermined angle so that the top part 26 of the culture vessel 25 is on the top and the bottom part 28 of the culture vessel 25 is on the bottom. An inclination angle α1 of the first flat culture vessel 25 with respect to the upper surface 32 of the sample holder 31 is in the range of 2 to 5°, preferably in the range of 2 to 3°.

In the stem cell culturing method, after confirming fixation of the first mesenchymal stem cells 35, by injecting a new culture medium 24 to the culture vessel 25 while discharging the culture medium 24 of the first flat culture vessel 25, proliferation of the stem cells 35 can be certainly promoted. In the stem cell culturing method, by inclining the first flat culture vessel 25 to the upper surface 32 of the sample holder 31 at the above-mentioned inclination angle, the first mesenchymal stem cells 35 and the culture medium 24 are inclined on the side of the top part 26 (or the side of the bottom part 26) of the culture vessel 25 in the first flat culture vessel 25, the water pressures of the first mesenchymal stem cells 35 and the culture medium 24 become large on the side of the top part 26 (or the side of the bottom part 26) of the culture vessel 25 and the first mesenchymal stem cells 35 are concentrated on the side of the bottom surface 36 of the culture vessel 25, whereby the activities of the first mesenchymal stem cells 35 are increased and the first mesenchymal stem cells 35 can be easily and quickly proliferated (differentiated) at the bottom surface 36 of the culture vessel 25.

The electron microscope 13 photographs an enlarged image of the planar shape of the first mesenchymal stem cells 35 in the first flat culture vessel 25 with intervals of about 1 to 2 hours, and the photographed enlarged image of the planar shape of the stem cells 35 is transmitted to the computer 11 with intervals of about 1 to 2 hours. The computer 11 stores (memorizes) the enlarged image of the planar shape of the first mesenchymal stem cells 35 and the photographing time transmitted from the electron microscope 13 in a storage area in the state of associating with the donor identifier (stem cells image storage means). The computer 11 displays the enlarged image of the planar shape of the first mesenchymal stem cells 35 and the photographing time transmitted from the electron microscope 13 on the display 16.

The person in charge confirms (views) the enlarged image of the planar shape of the first mesenchymal stem cells 35 displayed on the display 16 during 36 to 48 hours with intervals of about 1 to 2 hours, and judges whether or not the total surface area of the stem cells 25 reached the first target ratio (70 to 80% confluent) with respect to the bottom surface area of the culture vessel 25 while observing the total surface area of the stem cells 35 fixed to the bottom surface 36 of the first flat culture vessel 25 with respect to the bottom surface area of the culture vessel 25. Incidentally, the person in charge may directly observe the total surface area to the bottom surface area of the first flat culture vessel 25 of the first mesenchymal stem cells 35 from the observation window of the electron microscope 13 during 36 to 48 hours with intervals of about 1 to 2 hours, and may judge whether or not the total surface area of the stem cells 35 reached the first target ratio (70 to 80% confluent) with respect to the bottom surface area of the culture vessel 25.

As shown in Fig. 8, when the total surface area of the first mesenchymal stem cells 35 with respect to the bottom surface area of the first flat culture vessel 25 displayed on the display 16 is not reached the first target ratio (70 to 80% confluent) as a result of observation in the total surface area first observation step, the person in charge continuously observes the total surface area of the stem cells 35 with respect to the bottom surface area of the culture vessel 25 with intervals of about 1 to 2 hours. Incidentally, when the total surface area of the first mesenchymal stem cells 35 reached the first target ratio with respect to the total surface area of the enlarged image displayed on the display 16, it is judged that the total surface area of the stem cells 35 with respect to the bottom surface area of the first flat culture vessel 25 reached the first target ratio.

As a result of observation in the total surface area first observation step, the first mesenchymal stem cells 35 are proliferated on the bottom surface 36 (the bottom wall inner surface) of the first flat culture vessel 25, the stem cells 35 form colonies and the planar shape of the stem cells 35 is expanded, and when the total surface area of the stem cells 35 with respect to the bottom surface area of the culture vessel 25 displayed on the display 16 reached the first target ratio (70 to 80% confluent) as shown in Fig. 9, the stem cell first extracting step of extracting the stem cells 35 from the culture vessel 25 is carried out. In the stem cell first extracting step, the first mesenchymal stem cells 35 proliferated (differentiated) in the first flat culture vessel 25 are extracted from the culture vessel 25.

The person in charge such as a doctor, a nurse and a researcher, etc., clicks the total surface area first observation termination button displayed on the display 16 after confirming that the total surface area of the first mesenchymal stem cells 35 with respect to the bottom surface area of the first flat culture vessel 25 reached the first target ratio. When the total surface area first observation termination button is clicked, the computer 11 displays a process fifth display screen (not shown in the drawing) on the display 16. On the process fifth display screen, the number 2 and the donor data are displayed, and at the same time, a bone marrow aspirate separation termination message, a bone marrow aspirate extraction termination message, a deformation second observation termination message, a total surface area first observation termination message, a stem cell first extraction button, a stem cell centrifugation button, a stem cell second extraction button, a deformation second observation button, a total surface area second observation button and a stem cell third extraction button are displayed.

The person in charge clicks the stem cell first extraction button on the process fifth display screen, and causes the barcode reader 12 to read the QR code of the first flat culture vessel 25. When the QR code is transmitted from the barcode reader 12 to the computer 11, the computer 11 compares the donor data (the donor data read to the memory) displayed on the display 16 and the donor data indicated by the QR code read by the barcode reader 12, and when these donor data are matched, the number 2 and the donor data are displayed on the display 16, and at the same time, a bone marrow aspirate separation termination message, a bone marrow aspirate extraction termination message, a deformation second observation termination message, a total surface area first observation termination message, a stem cells first extraction on-going message and a stem cells first extraction termination button are displayed on the display 16. When these donor data are not matched, the computer 11 displays an error message and a culture stop message on the display 16.

The person in charge discharges the culture medium 24 injected into the first flat culture vessel 25 at the time of the total surface area first observation step from the culture vessel 25 by utilizing a syringe or a pipette, and after washing inside of the culture vessel 25 with PBS, a trypsin solution aspirated by the syringe or the pipette is injected into the culture vessel 25. When the trypsin solution is injected into the first flat culture vessel 25, the first mesenchymal stem cells 35 fixed to the bottom surface 36 of the culture vessel 25 are peeled off from the bottom surface 36 by the trypsin solution and floated on the surface of the trypsin solution. The person in charge uses a pipette to aspirate the stem cells 35 and accommodates the stem cells 35 in the pipette.

In the stem cell culturing method, by observing the total surface area of the first mesenchymal stem cells 35 with intervals of about 1 to 2 hours, the total surface area of the stem cells 35 with respect to the bottom surface area of the first flat culture vessel 25 can be accurately confirmed, and it can be certainly grasped that the total surface area of the stem cells 27 reaches the first target ratio with respect to the bottom surface area of the culture vessel 25.

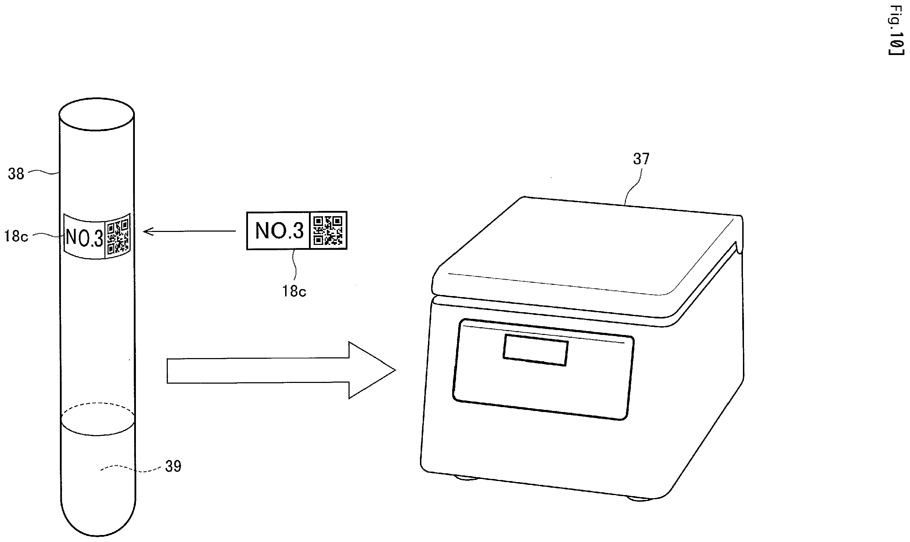

Fig. 10 is an explanation drawing showing an example of the stem cell centrifugation step. After the stem cell first extracting step in which the first mesenchymal stem cells 35 is extracted from the first flat culture vessel 25, the stem cell centrifugation step is carried out. In the stem cell centrifugation step, the first mesenchymal stem cells 35 extracted in the stem cell first extracting step are centrifuged into layers by a centrifugal separator 37. The person in charge such as a doctor, a nurse and a researcher, etc., aspirates the first mesenchymal stem cells 35 from the first flat culture vessel 25 by a pipette, and clicks the stem cells first extraction termination button displayed on the display 16.

When the stem cells first extraction termination button is clicked, the computer 11 displays a process sixth display screen (not shown in the drawing) on the display 16. On the process sixth display screen, the number 2 and the donor data are displayed, and at the same time, a bone marrow aspirate separation termination message, a bone marrow aspirate extraction termination message, a deformation first observation termination message, a total surface area first observation termination message, a stem cell first extraction termination message, a stem cell centrifugation button, a stem cell second extraction button, a deformation second observation button, a total surface area second observation button and a stem cell third extraction button are displayed.

The person in charge adheres the third code sheet 18c on which the QR code is printed to the outer circumferential surface of the glass test tube 38 into which the first mesenchymal stem cells 35 are injected. Next, the stem cell centrifugation button on the process sixth display screen is clicked to cause the barcode reader 12 to read the QR code of the glass test tube 38. When the QR code is transmitted from the barcode reader 12 to the computer 11, the computer 11 compares the donor data (the donor data read to the memory) displayed on the display 16 and the donor data indicated by the QR code read by the barcode reader 12, and when these donor data are matched, the number 3 and the donor data are displayed on the display 16, and at the same time, a bone marrow aspirate separation termination message, a bone marrow aspirate extraction termination message, a deformation first observation termination message, a total surface area first observation termination message, a stem cell first extraction termination message, a stem cell centrifugation on-going message and a stem cell centrifugation termination button are displayed on the display 16. When these donor data are not matched, the computer 11 displays an error message and a culture stop message on the display 16.

The person in charge injects (accommodates) the first mesenchymal stem cells 35 in a pipette into the glass test tube 38 and locates (sets) the glass test tube 38 in the centrifugal separator 37. The person in charge performs centrifugal separation of the first mesenchymal stem cells 35 for a predetermined time by the centrifugal separator 37 and then takes out the glass test tube 38 from the centrifugal separator 37. The first mesenchymal stem cells 35 in the glass test tube 38 are separated into two layers into layers in the vertical direction by the centrifugal separator 37.

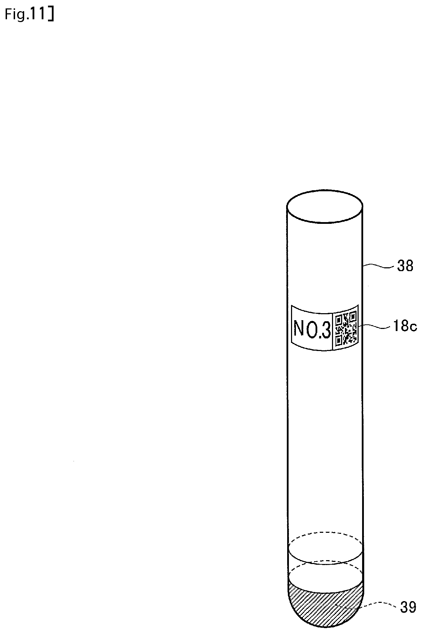

Fig. 11 is an explanation drawing showing an example of the stem cell second extracting step. After the stem cell centrifugation step in which the first mesenchymal stem cells 35 are separated into layers, the stem cell second extracting step is carried out. In the stem cell second extracting step, the second mesenchymal stem cells 39 positioned at the lower layer (the undermost layer) are extracted from the first mesenchymal stem cells 35 separated into layers. The person in charge such as a doctor, a nurse and a researcher, etc., performs separation of the first mesenchymal stem cells 35 into layers in the vertical direction by the centrifugal separator 37, then, the glass test tube 38 is taken out from the centrifugal separator 37, and a stem cell centrifugation termination button displayed on the display 16 is clicked.

When the stem cell centrifugation termination button is clicked, the computer 11 displays a process seventh display screen (not shown in the drawing) on the display 16. On the process seventh display screen, the number 3 and the donor data are displayed, and at the same time, a bone marrow aspirate separation termination message, a bone marrow aspirate extraction termination message, a deformation first observation termination message, a deformation first observation termination message, a stem cell first extraction termination message, a stem cell centrifugation termination message, a stem cell second extraction button, a deformation second observation button, a total surface area second observation button and a stem cell third extraction button are displayed.

The person in charge clicks the stem cell second extraction button on the process seventh display screen and causes the barcode reader 12 to read the QR code of the glass test tube 38. When the QR code is transmitted from the barcode reader 12 to the computer 11, the computer 11 compares the donor data (the donor data read to the memory) displayed on the display 16 and the donor data indicated by the QR code read by the barcode reader 12, and when these donor data are matched, the number 3 and the donor data are displayed on the display 16, and at the same time, a bone marrow aspirate separation termination message, a bone marrow aspirate extraction termination message, a deformation first observation termination message, a total surface area first observation termination message, a stem cell first extraction termination message, a stem cell centrifugation termination message, a stem cell second extraction on-going message and a stem cell second extraction termination message are displayed on the display 16. When these donor data are not matched, the computer 11 displays an error message and a culture stop message on the display 16.

The person in charge extracts the second mesenchymal stem cells 39 present in the lower layer (the undermost layer) of the first mesenchymal stem cells 35 separated into layers in the glass test tube 38. The person in charge extracts (sucks) the second mesenchymal stem cells 39 positioned at the lower layer (the undermost layer) of the first mesenchymal stem cells 35 separated into layers using a syringe. Or else, the second mesenchymal stem cells 39 positioned at the lower layer (the undermost layer) of the first mesenchymal stem cells 35 separated into layers are extracted (sucked) using a pipette.

In the stem cell culturing method, by centrifuging the first mesenchymal stem cells 35 containing unnecessary stem cells in the centrifugal separator 37 to separate it into layers in the vertical direction and extracting the second mesenchymal stem cells 39 positioned at the lower layer (the undermost layer) of the stem cells 35 centrifuged into layers, specific second mesenchymal stem cells 39 can be certainly extracted from the stem cells 35 and unnecessary mesenchymal stem cells can be removed from the stem cells 35.

When the stem cells 35 are proliferated with exceeding 80% of the total surface area of the first mesenchymal stem cells 35 with respect to the bottom surface area of the first flat culture vessel 25 (the first culturing vessel), the activity of the stem cells 35 is gradually lost, but in the stem cell culturing method, when the total surface area of the stem cells 35 is proliferated to 70 to 80% with respect to the bottom surface area of the culture vessel 25, the stem cells 35 are extracted from the culture vessel 25, so that the activity of the stem cells 35 can be retained and the stem cells 35 can be proliferated while maintaining the activity thereof, whereby the second mesenchymal stem cells 39 having activity can be extracted from the stem cells 35.

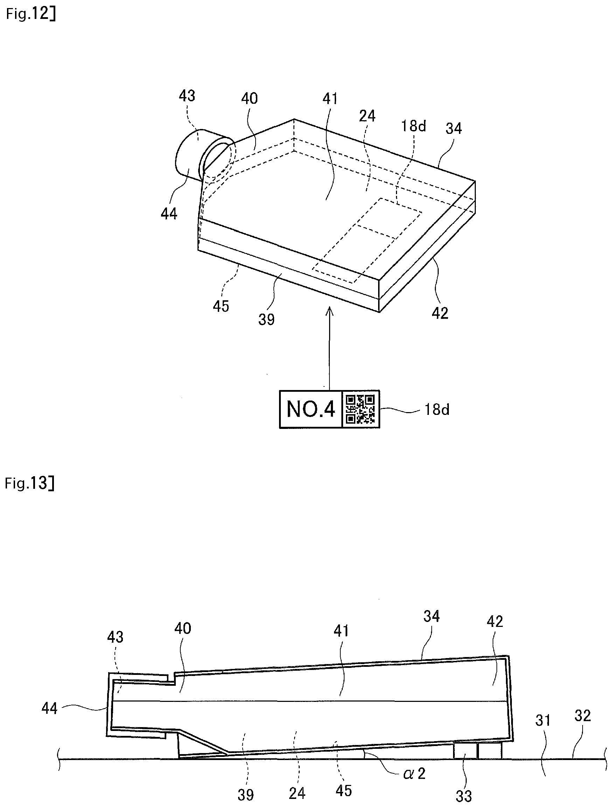

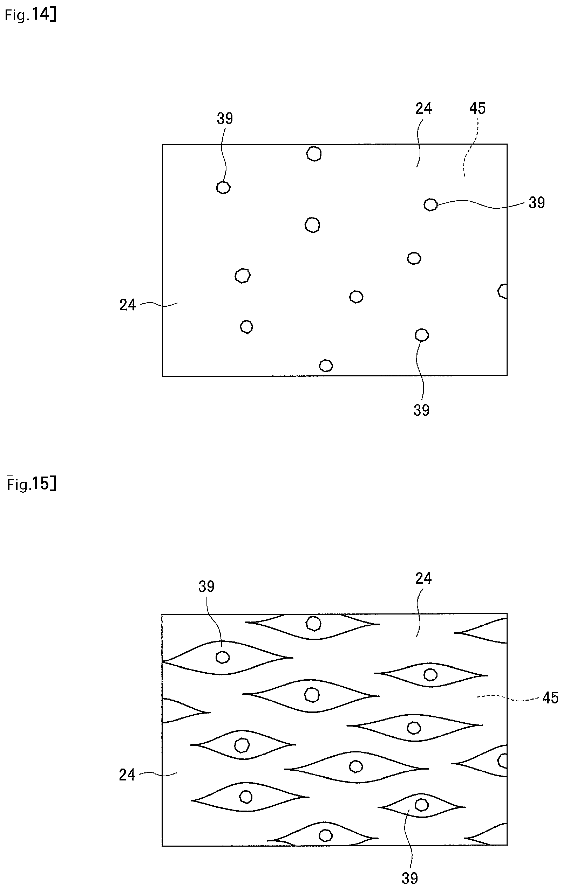

Fig. 12 is an explanation drawing showing an example of the deformation second observation step and Fig. 13 is a side view of the second flat culture vessel 25 (the second culturing vessel). Fig. 14 is a partial enlarged view showing an example of a planar shape of the second mesenchymal stem cells 39 and Fig. 15 is a partial enlarged view showing another example of a planar shape of the second mesenchymal stem cells 39. Figs. 14 and 15 show the enlarged images of the planar shapes of the second mesenchymal stem cells 39 photographed by the electron microscope 13.

After the stem cell second extracting step in which specific second mesenchymal stem cells 39 positioned at the lower layer (the undermost layer) are extracted from the first mesenchymal stem cells 35, the deformation second observation step is carried out. In the deformation second observation step, the second mesenchymal stem cells 39 and the culture medium 24 are injected (accommodated) into the second flat culture vessel 34 (the second culturing vessel) (the cell culture vessel), while statically allowing the culture vessel 34 at substantially the same temperature as the body temperature (about 37°C) for 36 to 48 hours (leave it quietly without moving it), deformation of the second mesenchymal stem cells 39 (the second stem cells) in the culture vessel 34 from an initial planar shape is observed by the electron microscope 13 during 36 to 48 hours with intervals of about 1 to 2 hours, and whether or not the stem cells 39 are fixed to the bottom surface 45 of the culture vessel 34 is judged. On the bottom surface 45 (the bottom wall outer surface) of the second flat culture vessel 34 into which the second mesenchymal stem cells 39 and the culture medium 24 are injected, a fourth code sheet 18d on which the QR code specifying the donor is printed is adhered.

The person in charge such as a doctor, a nurse and a researcher, etc., clicks a stem cell second extraction termination message displayed on the display 16 after extracting specific second mesenchymal stem cells 39 from the first mesenchymal stem cells 35. When the stem cell second extraction termination message is clicked, the computer 11 displays a process eighth display screen (not shown in the drawing) on the display 16. On the process eighth display screen, the number 4 and the donor data are displayed, and at the same time, a bone marrow aspirate separation termination message, a bone marrow aspirate extraction termination message, a deformation first observation termination message, a total surface area first observation termination message, a stem cell first extraction termination message, a stem cell centrifugation termination message, a stem cell second extraction termination message, a deformation second observation button, a total surface area second observation button and a stem cell third extraction button are displayed.

The person in charge adheres the fourth code sheet 18d on which the QR code is printed to the bottom surface 45 (the bottom wall outer surface) of the second flat culture vessel 34. Next, the deformation second observation button on the process eighth display screen is clicked to cause the barcode reader 12 to read the QR code of the culture vessel 34. When the QR code is transmitted from the barcode reader 12 to the computer 11, the computer 11 compares the donor data (the donor data read to the memory) displayed on the display 16 and the donor data indicated by the QR code read by the barcode reader 12, and when these donor data are matched, the number 4 and the donor data are displayed on the display 16, and at the same time, a bone marrow aspirate separation termination message, a bone marrow aspirate extraction termination message, a deformation first observation termination message, a total surface area first observation termination message, a stem cell first extraction termination message, a stem cell centrifugation termination message, a stem cell second extraction termination message, a deformation second observation message and a deformation second observation termination button are displayed on the display 16. When these donor data are not matched, the computer 11 displays an error message and a culture stop message on the display 16.

The person in charge injects (accommodates) the second mesenchymal stem cells 39 aspirated by a syringe or a pipette into the second flat culture vessel 34 and also injects (accommodates) the culture medium 24 aspirated by a syringe or a pipette into the culture vessel 34. The second flat culture vessel 34 (the second culturing vessel) used in the deformation second observation step is a flat vessel made of transparent glass or transparent plastic and having a bottom surface 45 with a small capacity and a predetermined area and having a substantially quadrangle planar shape, and the area of the bottom surface 45 is about 2-fold that of the first flat culture vessel 25 (the first culturing vessel). The second flat culture vessel 34 has a top part 40 and a bottom part 42, a central part 41 located between the top and bottom parts 40 and 42, and an injection port 43 formed at the top part 40. The injection port 43 is watertightly closed by a lid 44.

The second flat culture vessel 34 (the second culturing vessel) has a capacity of about 40 to 60 cc (preferably 50 cc), and its bottom surface area is about 50 to 72 mm². The second flat culture vessel 26 has a length of one side thereof of about 7 to 8.5 mm. Incidentally, as the second flat culture vessel 34, a flat vessel with a circular or elliptical in the planar shape and having a bottom surface 45 with a small capacity and a predetermined area may be used. The culture medium 24 is the same as that injected in the deformation first observation step. The second mesenchymal stem cells 39 injected into the culture vessel 34 are cultured with the culture medium 24 while being fixed on the bottom surface 45 of the culture vessel 34 with the lapse of time, and gradually proliferate (differentiate) on the bottom surface 45 of the culture vessel 34 to form colonies.

The person in charge removes the lid 44 from the injection port 43, injects (accommodates) the second mesenchymal stem cells 39 aspirated by a pipette into inside the culture vessel 34 from the injection port 43 of the second flat culture vessel 34, and injects (accommodates) the culture medium 24 aspirated by a syringe or a pipette into inside the culture vessel 34 from the injection port 43 of the culture vessel 34 and closes the injection port 43 by the lid 44. After injecting the second mesenchymal stem cells 39 and the culture medium 24 into the second flat culture vessel 34, the person in charge locates (sets) the culture vessel 34 in the sample holder 31 of the electron microscope 13.

A spacer 33 is interposed between the upper surface 32 of the sample holder 31 of the electron microscope 13 and the bottom part 42 of the second flat culture vessel 34, the bottom part 42 of the culture vessel 34 is held in a lifted state by the spacer 33, and the culture vessel 34 is kept in a state of being inclined at a predetermined angle so that the bottom part 42 of the culture vessel 34 is on the top and the top part 40 (the injection port 43) of the culture vessel 34 is on the bottom. Also, a spacer 33 is interposed between the upper surface 32 of the sample holder 31 of the electron microscope 13 and the top part 40 of the second flat culture vessel 34, the top part 40 of the culture vessel 34 is held in a lifted state by the spacer 33, the culture vessel 34 may be kept in a state of being inclined at a predetermined angle so that the top part 40 of the culture vessel 34 is on the top and the bottom part 42 of the culture vessel 34 is on the bottom. An inclination angle α2 of the second flat culture vessel 34 with respect to the upper surface 32 of the sample holder 31 is in the range of 2 to 5°, preferably in the range of 2 to 3°.

In the stem cell culturing method, by inclining the second flat culture vessel 34 to the upper surface 32 of the sample holder 31 at the above-mentioned inclination angle, the second mesenchymal stem cells 39 and the culture medium 24 are inclined on the side of the top part 40 (or the side of the bottom part 42) of the culture vessel 34 in the second flat culture vessel 34, the water pressures of the second mesenchymal stem cells 39 and the culture medium 24 become large on the side of the top part 40 (or the side of the bottom part 42) of the culture vessel 25 and the second mesenchymal stem cells 39 are concentrated on the side of the bottom surface 45 of the culture vessel 34, whereby the activities of the second mesenchymal stem cells 39 are increased and the second mesenchymal stem cells 39 can be easily and quickly fixed on the bottom surface 45 of the culture vessel 34.

The electron microscope 13 photographs an enlarged image of the planar shape of the second mesenchymal stem cells 39 injected into the second flat culture vessel 34 with intervals of about 1 to 2 hours, and the photographed enlarged image of the planar shape of the stem cells 39 is transmitted to the computer 11 with intervals of about 1 to 2 hours. The computer 11 stores (memorizes) the enlarged image of the planar shape of the second mesenchymal stem cells 39 and the photographing time transmitted from the electron microscope 13 in a storage area in the state of associating with the donor identifier (stem cells image storage means). The computer 11 displays the enlarged image of the planar shape of the second mesenchymal stem cells 39 and the photographing time transmitted from the electron microscope 13 on the display 16. The person in charge confirms (views) the enlarged image of the planar shape of the second mesenchymal stem cells 39 displayed on the display 16 during 36 to 48 hours with intervals of about 1 to 2 hours, and observes change in the planar shape of the stem cells 39. Incidentally, the person in charge may directly observe change in the planar shape of the second mesenchymal stem cells 39 from the observation window of the electron microscope 13 during 36 to 48 hours with intervals of about 1 to 2 hours.

The initial planar shape of the second mesenchymal stem cells 39 is substantially circular, and when the planar shape of the stem cells 39 is substantially circular, the stem cells 39 are not fixed to the bottom surface 45 (the bottom wall inner surface) of the second flat culture vessel 34, and proliferation (differentiation) of the stem cells 39 is not started. The planar shape of the second mesenchymal the stem cells 39 after deformation is a flat shape in which the stem cells 39 are extended in irregular in one direction with substantially the circular as a core, and the stem cells 39 are fixed to the bottom surface 45 (the bottom wall inner surface) of the second flat culture vessel 34 and proliferation (differentiation) of the stem cells 39 are started.

As shown in Fig. 14, when the planar shape of the second mesenchymal stem cells 39 displayed on the display 16 is observed to remain substantially circular as a result of observation in the deformation second observation step, the person in charge judges that the stem cells 39 are not fixed to the bottom surface 45 (the bottom wall inner surface) of the second flat culture vessel 34, and continuously observes changes in the planar shape of the stem cells 39 with intervals of about 1 to 2 hours. As shown in Fig. 15, when the planar shape of the second mesenchymal stem cells 39 displayed on the display 16 is deformed from substantially circular into an irregular flat shape with substantially the circular as a core as a result of observation in the deformation second observation step, the person in charge judges that the stem cells 39 is fixed to the bottom surface 45 of the second flat culture vessel 34.

If a large culture vessel having a capacity exceeding 60 cc and the bottom surface area exceeding 72 mm² is used at the time of fixing the second mesenchymal stem cells 39, the stem cells 39 tend to be difficultly fixed to the bottom surface of the vessel and proliferation of the stem cells 39 becomes slow, but in the stem cell culturing method, the stem cells 39 can be easily fixed to the bottom surface 45 of the culture vessel 34 by using the second flat culture vessel 34 having the above-mentioned capacity and the above-mentioned bottom surface area, and the stem cells 39 can be quickly proliferated in the culture vessel 34.

In the stem cell culturing method, since deformation of the second mesenchymal stem cells 39 in the culture vessel 34 from an initial planar shape is observed during 36 to 48 hours with intervals of about 1 to 2 hours while statically allowing the second flat culture vessel 34 at substantially the same temperature as the body temperature for 36 to 48 hours, deformation of the stem cells 39 is never overlooked and fixation of the stem cells 39 to the bottom surface 45 of the culture vessel 34 can be accurately confirmed.

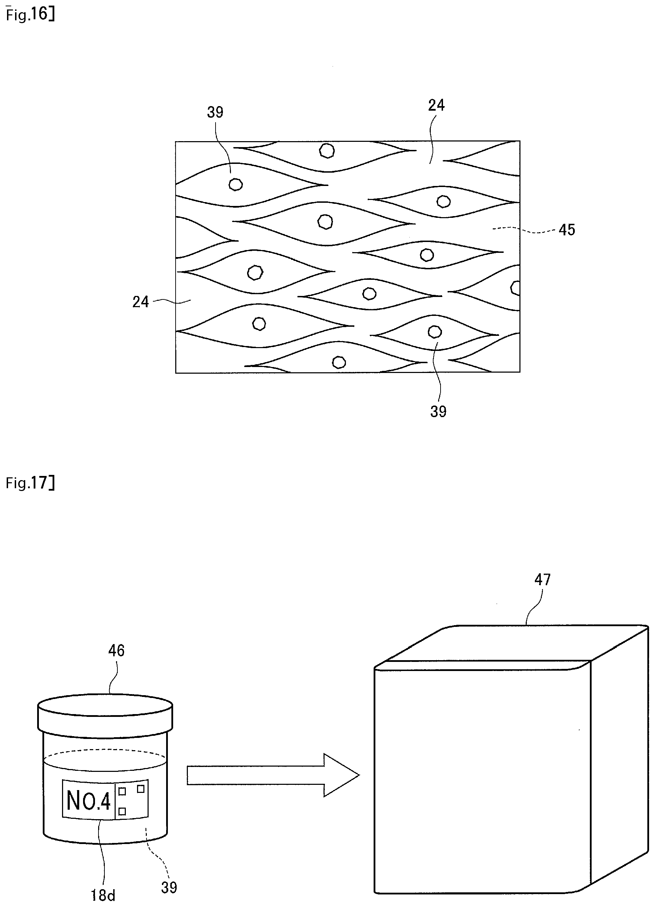

Fig. 16 is a partial enlarged view showing another example of a planar shape of the second mesenchymal stem cells 39 and Fig. 17 is a drawing showing an example of preservation of the second mesenchymal stem cells 39. Fig. 16 shows the enlarged image of the planar shape of the second mesenchymal stem cells 39 photographed by the electron microscope 13. As a result of observation in the deformation second observation step, the second mesenchymal stem cells 39 (the second stem cells) is deformed from a substantially circular shape (an initial planar shape) to an irregular flat shape with substantially the circular as a core, and after stem cells third observation step confirming fixation of the stem cells 39 to the bottom surface 45 of the second flat culture vessel 34 (the second culturing vessel), the total surface area second observation step is carried out.

In the total surface area second observation step, the culture medium 24 injected into the second flat culture vessel 34 is discharged from the culture vessel 34, and a new culture medium 24 is injected (accommodated) into the culture vessel 34. Next, the total surface area of the second mesenchymal stem cells 39 fixed to the bottom surface 45 of the culture vessel 34 with respect to the bottom surface area of the culture vessel 34 is observed by the electron microscope 13 during 36 to 48 hours with intervals of about 1 to 2 hours while statically allowing (leave it quietly without moving it) the second flat culture vessel 34 at substantially the same temperature as the body temperature (about 36 to 37°C) for 36 to 48 hours, and it is judged whether or not the total surface area of the stem cells 39 reached the second target ratio with respect to the bottom surface area of the culture vessel 34. The second target ratio of the total surface area of the second mesenchymal stem cells 39 with respect to the bottom surface area of the second flat culture vessel 34 is 88 to 92% (88 to 92% confluent).

The person in charge such as a doctor, a nurse and a researcher, etc., clicks the deformation second observation termination button displayed on the display 16 after confirming fixation of the second mesenchymal stem cells 39 to the bottom surface 45 of the second flat culture vessel 34. When the deformation second observation termination button is clicked, the computer 11 displays a process ninth display screen (not shown in the drawing) on the display 16. On the process ninth display screen, the number 4 and the donor data are displayed, and at the same time, a bone marrow aspirate separation termination message, a bone marrow aspirate extraction termination message, a deformation first observation termination message, a total surface area first observation termination message, a stem cell first extraction termination message, a stem cell centrifugation termination message, a stem cell second extraction termination message, a deformation second observation termination message, a total surface area second observation button and a stem cell third extraction button are displayed.

The person in charge clicks the total surface area second observation button on the process ninth display screen to remove the second flat culture vessel 34 from the sample holder 31 of the electron microscope 13, and causes the barcode reader 12 to read the QR code of the culture vessel 34. When the QR code is transmitted from the barcode reader 12 to the computer 11, the computer 11 compares the donor data (the donor data read to the memory) displayed on the display 16 and the donor data indicated by the QR code read by the barcode reader, and when these donor data are matched, the number 4 and the donor data are displayed on the display 16, and at the same time, a bone marrow aspirate separation termination message, a bone marrow aspirate extraction termination message, a deformation first observation termination message, a total surface area first observation termination message, a stem cell first extraction termination message, a stem cell centrifugation termination message, a stem cell second extraction termination message, a deformation second observation termination message, a total surface area second observation on-going message and a total surface area second observation termination button are displayed on the display 16. When these donor data are not matched, the computer 11 displays an error message and a culture stop message on the display 16.

The person in charge discharges the culture medium 24 injected into the second flat culture vessel 34 in the total surface area second observation step from the culture vessel 34 utilizing a syringe or a pipette and injects (accommodates) a new culture medium 24 aspirated by a syringe or a pipette into the culture vessel 34. The new culture medium 24 is the same as that injected in the deformation first observation step. The person in charge locates (sets) the culture vessel 34 at the sample holder 31 of the electron microscope 13 after injecting the new culture medium 24 into the second flat culture vessel 34.

A spacer 33 is interposed between the upper surface 32 of the sample holder 31 of the electron microscope 13 and the bottom part 42 of the second flat culture vessel 34, the bottom part 42 of the culture vessel 34 is held in a lifted state by spacer 33, and the culture vessel 34 is kept in a state of being inclined at a predetermined angle so that the bottom part 42 of the culture vessel 34 is on the top and the top part 40 (the injection port 43) of the culture vessel 34 is on the bottom (see Fig. 13). Also, the spacer 33 is interposed between the upper surface 32 of the sample holder 31 of the electron microscope 13 and the top part 40 of the second flat culture vessel 34, the top part 40 of the culture vessel 34 is held in a lifted state by the spacer 33, and the culture vessel 34 may be kept in a state of being inclined at a predetermined angle so that the top part 40 of the culture vessel 34 is on the top and the bottom part 42 of the culture vessel 34 is on the bottom. An inclination angle α2 of the second flat culture vessel 34 with respect to the upper surface 32 of the sample holder 31 is in the range of 2 to 5°, preferably in the range of 2 to 3°.

In the stem cell culturing method, after confirming fixation of the second mesenchymal stem cells 39, by injecting a new culture medium 24 into the culture vessel 34 while discharging the culture medium 24 of the second flat culture vessel 34, proliferation of the stem cells 39 can be certainly promoted. In the stem cell culturing method, by inclining the second flat culture vessel 34 to the upper surface 32 of the sample holder 31 at the above-mentioned inclination angle, the second mesenchymal stem cells 39 and the culture medium 24 are inclined on the side of the top part 40 (or the side of the bottom part 42) of the culture vessel 34 in the second flat culture vessel 34, and on the side of the top part 40 (or the side of the bottom part 42) of the culture vessel 34, the water pressures of the second mesenchymal stem cells 39 and the culture medium 24 become large and the second mesenchymal stem cells 39 are concentrated on the side of the bottom surface 45 of the culture vessel 34, whereby the activities of the second mesenchymal stem cells 39 are increased and the second mesenchymal stem cells 39 can be easily and quickly proliferated (differentiated) on the bottom surface 45 of the culture vessel 34.

The electron microscope 13 photographs an enlarged image of the planar shape of the second mesenchymal stem cells 39 in the second flat culture vessel 34 with intervals of about 1 to 2 hours, and the photographed enlarged image of the planar shape of the stem cells 39 is transmitted to the computer 11 with intervals of about 1 to 2 hours. The computer 11 stores (memorizes) the enlarged image of the planar shape of the second mesenchymal stem cells 39 and the photographing time transmitted from the electron microscope 13 in a storage area in the state of associating with the donor identifier (stem cells image storage means). The computer 11 displays the enlarged image of the planar shape of the second mesenchymal stem cells 39 and the photographing time transmitted from the electron microscope 13 on the display 16.

The person in charge confirms (views) the enlarged image of the planar shape of the second mesenchymal stem cells 39 displayed on the display 16 during 36 to 48 hours with intervals of about 1 to 2 hours, and judges whether or not the total surface area of the stem cells 39 reached the second target ratio (82 to 92% confluent) with respect to the bottom surface area of the culture vessel 34 while observing the total surface area of the stem cells 39 fixed to the bottom surface 45 of the second flat culture vessel 34 with respect to the bottom surface area of the culture vessel 34. Incidentally, the person in charge may directly observe the total surface area of the second flat culture vessel 34 of the second mesenchymal stem cells 39 with respect to the bottom surface area from the observation window of the electron microscope 13 during 36 to 48 hours with intervals of about 1 to 2 hours, and may judge whether or not the total surface area of the stem cells 39 reached the second target ratio (88 to 92% confluent) with respect to the bottom surface area of the culture vessel 34.

As shown in Fig. 15, when the total surface area of the second mesenchymal stem cells 39 displayed on the display 16with respect to the bottom surface area of the second flat culture vessel 34 is not reached the second target ratio (88 to 92% confluent) as a result of observation in the total surface area second observation step, the person in charge continuously observes the total surface area of the stem cells 39 with respect to the bottom surface area of the culture vessel 34 with intervals of about 1 to 2 hours. Incidentally, when the total surface area of the second mesenchymal stem cells 39 reached the second target ratio with respect to the total surface area of the enlarged image displayed on the display 16, it is judged that the total surface area of the stem cells 39 with respect to the bottom surface area of the second flat culture vessel 34 reached the second target ratio.

As a result of observation in the total surface area second observation step, the second mesenchymal stem cells 39 are proliferated on the bottom surface 45 (the bottom wall inner surface) of the second flat culture vessel 34 (the second culturing vessel), the stem cells 39 form colonies and the planar shape of the stem cells 39 is expanded, and when the total surface area of the stem cells 39 with respect to the bottom surface area of the culture vessel 34 displayed on the display 16 reached the second target ratio (88 to 92% confluent) as shown in Fig. 16, the stem cell third extracting step of extracting the stem cells 39 from the culture vessel 34 is carried out. In the stem cell third extracting step, the second mesenchymal stem cells 39 proliferated (differentiated) in the second flat culture vessel 34 are extracted from the culture vessel 34. In the stem cell culturing method, by observing the total surface area with intervals of about 1 to 2 hours, the total surface area of the second mesenchymal stem cells 39 with respect to the bottom surface area of the second flat culture vessel 34 can be accurately confirmed and it can be certainly grasped that the total surface area of the stem cells 39 has reached the second target ratio with respect to the bottom surface area of the culture vessel 34.

The person in charge such as a doctor, a nurse and a researcher, etc., clicks the total surface area second observation termination button displayed on the display 16 after confirming that the total surface area of the second mesenchymal stem cells 39 with respect to the bottom surface area of the second flat culture vessel 34 reached the second target ratio. The total surface area second observation termination button is clicked, the computer 11 displays a process tenth display screen (not shown in the drawing) on the display 16. On the process tenth display screen, the number 4 and the donor data are displayed, and at the same time, a bone marrow aspirate separation termination message, a bone marrow aspirate extraction termination message, a deformation first observation termination message, a total surface area first observation termination message, a stem cell first extraction termination message, a stem cell centrifugation termination message, a stem cell second extraction termination message, a deformation second observation termination message, a total surface area second observation termination message and a stem cell third extraction button are displayed.

The person in charge clicks the stem cell third extraction button on the process tenth display screen and causes the barcode reader 12 to read the QR code of the second flat culture vessel 34. When the QR code is transmitted from the barcode reader 12 to the computer 11, the computer 11 compares the donor data (the donor data read to the memory) displayed on the display 16 and the donor data indicated by the QR code read by the barcode reader 12, and when these donor data are matched, the number 4 and the donor data are displayed on the display 16, and at the same time, a bone marrow aspirate separation termination message, a bone marrow aspirate extraction termination message, a deformation first observation termination message, a total surface area first observation termination message, a stem cell first extraction termination message, a stem cell centrifugation termination message, a stem cell second extraction termination message, a deformation second observation termination message, a total surface area second observation termination message and a stem cell third extraction termination button are displayed on the display 16. When these donor data are not matched, the computer 11 displays an error message and a culture stop message on the display 16.

In the total surface area second observation step, the person in charge discharges the culture medium 24 injected into the second flat culture vessel 34 from the culture vessel 34 by utilizing a syringe or a pipette, and after washing inside of the culture vessel 34 with PBS, a trypsin solution aspirated by a syringe or a pipette is injected into the culture vessel 34. When the trypsin solution is injected into the second flat culture vessel 34, the second mesenchymal stem cells 39 fixed to the bottom surface 45 of the culture vessel 34 are peeled off from the bottom surface 45 by the trypsin solution and floated on the surface of the trypsin solution.

The person in charge uses a pipette to aspirate the second mesenchymal stem cells 39 and injects (accommodates) the stem cells 39 into a preserving vessel 46 from a pipette. The second mesenchymal stem cells 39 injected into the preserving vessel 46 are a specific type (substantially single kind) of pure mesenchymal stem cells having activity with an object to be cultured from which unnecessary mesenchymal stem cells have been removed. After injecting the second mesenchymal stem cells 39 into the preserving vessel 46 from a pipette, the person in charge adheres the fourth code sheet 18d on which the QR code is printed to the outer circumferential surface of the preserving vessel 46.

The person in charge clicks a stem cell third extraction termination button on the process tenth display screen displayed on the display 16, makes the barcode reader 12 read the QR code of the preserving vessel 46, and then the preserving vessel 46 into which the second mesenchymal stem cells 39 are injected is stirred in a refrigerator 47. The second mesenchymal stem cells 39 are preserved in the refrigerator 47 at about 3 to 4°C.

When the QR code is transmitted from the barcode reader 12 to the computer 11, the computer 11 compares the donor data (the donor data read to the memory) displayed on the display 16 and the donor data indicated by the QR code read by the barcode reader 12, and when these donor data are matched, the number 4 and the donor data are displayed on the display 16, and at the same time, a bone marrow aspirate separation termination message, a bone marrow aspirate extraction termination message, a deformation first observation termination message, a total surface area first observation termination message, a stem cell first extraction termination message, a stem cell centrifugation termination message, a stem cell second extraction termination message, a deformation second observation termination message, a total surface area second observation termination message and a stem cell third extraction termination message are displayed on the display 16.

If the stem cells 39 are proliferated with exceeding 92% of the total surface area of the second mesenchymal stem cells 39 with respect to the bottom surface area of the second flat culture vessel 34 (the second culturing vessel), the activity of the stem cells 39 is gradually lost, but in the stem cell culturing method, when the total surface area of the stem cells 39 is proliferated to 88 to 92% with respect to the bottom surface area of the culture vessel 34, the stem cells 39 are extracted from the culture vessel 34, so that the activity of the stem cells 39 can be retained and the stem cells 39 can be proliferated while maintaining the activity thereof.

In the stem cell culturing method, the second bone marrow aspirate 23 positioned at an intermediate layer is extracted among the first bone marrow aspirate 19 separated into layers, the second bone marrow aspirate 23 is cultured with the culture medium 24 to fix the first mesenchymal stem cells 35 (the first stem cells) to the bottom surface 36 of the first flat culture vessel 25 (the first culturing vessel) and also the first mesenchymal stem cells 35 are proliferated. When the total surface area of the first mesenchymal stem cells 35 reached the first target ratio with respect to the bottom surface area of the first flat culture vessel 25, the first mesenchymal stem cells 35 are extracted from the culture vessel 25, the second mesenchymal stem cells 39 (the second stem cells) positioned at the lower layer (the undermost layer) among the first mesenchymal stem cells 35 centrifuged into layers are extracted, the second mesenchymal stem cells 39 are cultured with the culture medium 24 to fix the second mesenchymal stem cells 39 to the bottom surface 45 of the second flat culture vessel 34 and the second mesenchymal stem cells 39 are proliferated. When the total surface area of the second mesenchymal stem cells 39 reached the second target ratio with respect to the bottom surface area of the second flat culture vessel 34, the second mesenchymal stem cells 39 are extracted from the culture vessel 34. Thus, by extracting a specific second bone marrow aspirate 23 from the first bone marrow aspirate 19 separated into layers and by extracting specific second mesenchymal the stem cells 39 from the first mesenchymal stem cells 35 separated into layers, proliferation of various stem cells can be prevented and only specific types of stem cells (the second mesenchymal stem cells) which are an object to be produced can be cultured, and pure (genuine) stem cells from which unnecessary stem cells had been removed can be produced.

The stem cell culturing method can culture only specific types of stem cells (the second mesenchymal stem cells 39), so that the stem cells having great therapeutic effect to various diseases and regenerative effect in regenerative medicine and having high possibility of completely curing various diseases as well as high possibility of regenerating various tissues or various organs can be cultured.

### Explanation of reference numerals

- 10: Stem cells culturing system
- 11: Computer
- 12: Barcode reader
- 13: Electron microscope
- 14: Keyboard
- 15: Mouse
- 16: Display
- 18a: First code sheet
- 18b: Second code sheet
- 18c: Third code sheet
- 18d: Fourth code sheet
- 19: First bone marrow aspirate
- 20: Glass test tube
- 21: Test tube stand
- 22: Thermostat chamber
- 23: Second bone marrow aspirate
- 24: Culture medium
- 25: First flat culture vessel (First culturing vessel)
- 26: Top part
- 27: Central part
- 28: Bottom part
- 29: Injection port
- 30: Lid
- 31: Sample holder
- 32: Upper surface
- 33: Spacer
- 34: Second flat culture vessel (Second culturing vessel)
- 35: First mesenchymal stem cells (First stem cells)
- 36: Bottom surface
- 37: Centrifugal separator
- 38: Glass test tube
- 39: Second mesenchymal stem cells (Second stem cells)
- 40: Top part
- 41: Central part
- 42: Bottom part
- 43: Injection port
- 44: Lid
- 45: Bottom surface
- 46: Preserving vessel
- 47: Refrigerator

## Claims

1. A stem cell culturing method for culturing specific types of stem cells using a first bone marrow aspirate collected from a donor, wherein
the stem cell culturing method comprises a bone marrow aspirate separating step of separating the first bone marrow aspirate collected from a donor into layers, a bone marrow aspirate extracting step of extracting a second bone marrow aspirate positioned at an intermediate layer among the first bone marrow aspirate separated into layers in the bone marrow aspirate separating step, a stem cell first extracting step of injecting the second bone marrow aspirate extracted in the bone marrow aspirate extracting step and a predetermined culture medium into a first culturing vessel having a predetermined volume and a predetermined area of a bottom surface, statically allowing the first culturing vessel for a predetermined time to fix the first stem cells to the bottom surface of the first culturing vessel and proliferating the first stem cells, and when a total surface area of the first stem cells with respect to the bottom surface of the first culturing vessel reached a first target ratio by expanding a planar shape of the first stem cells at the bottom surface area of the first culturing vessel, extracting the first stem cells from the first culturing vessel, a stem cell centrifugation step of centrifuging the first stem cells extracted in the stem cell first extracting step into layers, a stem cell second extracting step of extracting the second stem cells positioned at an undermost layer after separating the first stem cells into layers in the stem cell centrifugation step, and a stem cell third extracting step of injecting the second stem cells extracted in the stem cell second extracting step and a predetermined culture medium into a second culturing vessel having a larger capacity and a larger bottom surface area of the bottom surface than those of the first culturing vessel, statically allowing the second culturing vessel for a predetermined time to fix the second stem cells to the bottom surface of the second culturing vessel and proliferating the second stem cells, and when a total surface area of the second stem cells with respect to the bottom surface of the second culturing vessel reached a second target ratio by expanding a planar shape of the second stem cells at the bottom surface area of the second culturing vessel, extracting the second stem cells from the second culturing vessel.

2. The stem cell culturing method according to claim 1, wherein the stem cell culturing method comprises a deformation first observation step of injecting the second bone marrow aspirate extracted in the bone marrow aspirate extracting step and the culture medium into the first culturing vessel, then, observing deformation of the first stem cells in the first culturing vessel from an initial planar shape while statically allowing it for a predetermined time statically in a state of inclining the first culturing vessel at a predetermined angle with predetermined time intervals, and a total surface area first observation step of injecting a new culture medium into the first culturing vessel while discharging the culture medium in the first culturing vessel by judging the first stem cells be fixed to the bottom surface of the first culturing vessel when the first stem cells are deformed from the initial planar shape to a predetermined planar shape as a result of observation in the deformation first observation step, and observing a total surface area of the first stem cells fixed to the bottom surface of the first culturing vessel with respect to the bottom surface area of the first culturing vessel while statically allowing it for a predetermined time in a state of inclining the first culturing vessel at a predetermined angle with predetermined time intervals, and in the stem cell first extracting step, when the planar shape of the first stem cells is expanded by proliferating the first stem cells and the total surface area of the first stem cells with respect to the bottom surface area of the first culturing vessel reached the first target ratio as a result of observation in the total surface area first observation step, the first stem cells are extracted from the first culturing vessel.

3. The stem cell culturing method according to claim 2, wherein the stem cell culturing method comprises a deformation second observation step of injecting the second stem cells extracted in the stem cell second extracting step and the culture medium into the second culturing vessel, then, observing deformation of the second stem cells in the second culturing vessel from an initial planar shape while statically allowing it in a state of inclining the second culturing vessel at a predetermined angle for a predetermined time statically with predetermined time intervals, and a total surface area second observation step of injecting a new culture medium into the second culturing vessel while discharging the culture medium in the second culturing vessel by judging the second stem cells be fixed to the bottom surface of the second culturing vessel when the second stem cells are deformed from the initial planar shape to a predetermined planar shape as a result of observation in the deformation second observation step, and observing a total surface area of the second stem cells fixed to the bottom surface of the second culturing vessel with respect to the bottom surface area of the second culturing vessel while statically allowing it for a predetermined time in a state of inclining the second culturing vessel at a predetermined angle with predetermined time intervals, and in the stem cell third extracting step, when the planar shape of the second stem cells is expanded by proliferating the second stem cells and the total surface area of the second stem cells with respect to the bottom surface area of the second culturing vessel reached the second target ratio as a result of observation in the total surface area second observation step, the second stem cells are extracted from the second culturing vessel.

4. The stem cell culturing method according to any one of claim 1 to claim 3, wherein, in the bone marrow aspirate separating step, 2 to 3 cc of the first bone marrow aspirate are collected from the donor, the 2 to 3 cc of the first bone marrow aspirate are injected into a separating vessel extending in the vertical direction, and the separating vessel is statically allowed at substantially the same temperature as a body temperature for a predetermined time to separate the first bone marrow aspirate in the separating vessel into layers in the vertical direction, and in the bone marrow aspirate extracting step, the second bone marrow aspirate positioned at an intermediate layer in the first bone marrow aspirate separated into layers in the separating vessel is extracted.

5. The stem cell culturing method according to any one of claim 2 to claim 4, wherein a capacity of the first culturing vessel is about 20 to 30 cc, an initial planar shape of the first stem cells is substantially circular, a planar shape of the first stem cells after deformation is a flat shape in which the first stem cells are extended in irregular in one direction with substantially the circular as a core, and in the deformation first observation step, deformation of the first stem cells in the first culturing vessel from an initial planar shape is observed during 12 to 24 hours with intervals of about 1 to 2 hours while statically allowing the first culturing vessel at substantially the same temperature as a body temperature for 12 to 24 hours, and when the first stem cells are deformed in an irregular flat shape, the first stem cells are judged to be fixed to the bottom surface of the first culturing vessel.

6. The stem cell culturing method according to any one of claim 1 to claim 5, wherein, in the total surface area first observation step, the total surface area of the first stem cells fixed to the bottom surface of the first culturing vessel with respect to the bottom surface area of the first culturing vessel is observed during 36 to 48 hours with intervals of about 1 to 2 hours while statically allowing the first culturing vessel at substantially the same temperature as a body temperature for 36 to 48 hours.

7. The stem cell culturing method according to any one of claim 1 to claim 6, wherein the first target ratio of the total surface area of the first stem cells with respect to the bottom surface area of the first culturing vessel is 70 to 80%.

8. The stem cell culturing method according to any one of claim 1 to claim 7, wherein, in the stem cell centrifugation step, the first stem cells is injected into the separating vessel, the separating vessel is located in a centrifugal separator to centrifuge the first stem cells, and in the stem cell second extracting step, the first stem cells are centrifuged into layers in the separating vessel, and then, the second stem cells located at the undermost layer are extracted.

9. The stem cell culturing method according to any one of claim 3 to claim 8, wherein a capacity of the second culturing vessel is about 40 to 60 cc, an initial planar shape of the second stem cells is substantially circular, a planar shape of the first stem cells after deformation of the second stem cells is a flat shape in which the second stem cells are extended in irregular in one direction with substantially the circular as a core, and in the deformation second observation step, deformation of the second stem cells in the second culturing vessel from an initial planar shape is observed during 36 to 48 hours with intervals of about 1 to 2 hours while statically allowing the second culturing vessel at substantially the same temperature as a body temperature for 36 to 48 hours, and when the second stem cells are deformed in an irregular flat shape, the second stem cells are judged to be fixed to the bottom surface of the second culturing vessel.

10. The stem cell culturing method according to any one of claim 1 to claim 9, wherein, in the total surface area second observation step, the total surface area of the second stem cells fixed to the bottom surface of the second culturing vessel with respect to the bottom surface area of the second culturing vessel is observed during the 36 to 48 hours with intervals of about 1 to 2 hours while statically allowing the second culturing vessel at substantially the same temperature as a body temperature for 36 to 48 hours.

11. The stem cell culturing method according to any one of claim 1 to claim 10, wherein the second target ratio of the total surface area of the second stem cells with respect to the bottom surface area of the second culturing vessel is 88 to 92%.

12. The stem cell culturing method according to any one of claim 1 to claim 11, wherein the first and the second stem cells are mesenchymal stem cells.
